# EUROPEAN PATENT APPLICATION

(11) **EP 4 529 929 A1**
(43) Date of publication of application: **02.04.2025**
(21) Application number: 24202817.3
(22) Date of filing: 26.09.2024
(51) Int. Cl.: A61K 39/00, C07K 14/725

(54) **ISOLATED NUCLEIC ACID MOLECULE COMPRISING NKP30 TRANSMEMBRANE DOMAIN AND CHIMERIC ANTIGEN RECEPTOR COMPRISING THE SAME**

(30) Priority: 28.09.2023 US 202363540954 P
(71) Applicant: Pell Bio-Med Technology Co., Ltd., Taipei City (TW)
(72) Inventor: HSU, Felix, Taipei City (TW); LIN, Wei-Chi, Taipei City (TW); WU, Wen-Ting, Taipei City (TW); LIN, Chen-Lung, Taipei City, (TW)
(74) Representative: Cabinet Chaillot

(57) **Abstract**

Provided is an isolated nucleic acid molecule comprising a NKp30 transmembrane domain, and a chimeric antigen receptor comprising the same. The isolated nucleic acid molecule comprising a NKp30 transmembrane domain comprises nucleic acid sequences encoding (a) an extracellular antigen binding domain, comprising a heavy chain variable region; (b) a hinge domain; (c) a NKp30 transmembrane domain; and (d) a NKp30 cytoplasmic domain. By introducing nucleic acid sequences of the NKp30 transmembrane domain and the NKp30 cytoplasmic domain in combination with the extracellular antigen binding domain into a T cell, the resulting CAR-T cell is a multi-chain CAR-T cell with a NKp30 receptor complex. Consequently, the resulting CAR-T cell forms stable immune synapses with cancer cells and exhibits excellent cytotoxicity against cancer cells.

## Description

The present disclosure relates to a nucleic acid molecule for preparing a chimeric antigen receptor (CAR), particularly, a nucleic acid molecule for preparing CAR to exhibit excellent cytotoxicity against cancer cells. The present disclosure also relates to a CAR comprising the nucleic acid molecule and applications of the CAR comprising the nucleic acid molecule.

Cancer is caused by multi-stage transformation from normal cells to tumor cells and usually starts from a milder pre-cancerous lesion which gradually progresses to a malignant tumor, thereby leading to metastasis. The foresaid transformation is not caused by a single factor, but a result of interactions between individual genetic factors and external factors.

Cancer is the second leading cause of death in the world. According to the statistics from the World Health Organization in 2020, nearly ten million people die of cancer worldwide, accounting for one-sixth of the global deaths. Among all, the most common cancers in men are lung cancer, prostate cancer, colon cancer, gastric cancer and liver cancer; and the most common cancers in women are breast cancer, colorectal cancer, lung cancer, cervical cancer and thyroid cancer.

Cancer not only causes physical and mental pain to the patient, but also brings severe emotional and financial stress to the patient's family. However, in the high-stressed environment of the modern society, it is difficult to completely eliminate the adverse external factors, and the number of cancer patients still increases year by year.

Accordingly, there remains an urgent need for an effective treatment for cancer.

For overcoming the problems in the prior art, one objective or advantage of the present disclosure is to provide a novel chimeric antigen receptor (CAR) design for CAR-T cell, and when they are introduced into the human body, the modified T cells with specific antigen binding domains are able to identify tumor cells expressing that antigen, followed by killing the tumor cells through cytotoxicity.

To achieve the foresaid objective, the present disclosure provides an isolated nucleic acid molecule comprising nucleic acid sequences encoding the following parts: (a) an extracellular antigen binding domain, which comprises a heavy chain variable region; (b) a hinge domain; (c) a Natural Killer Protein 30 (NKp30) transmembrane domain; and (d) a NKp30 cytoplasmic domain.

By introducing nucleic acid sequences of the NKp30 transmembrane domain and the NKp30 cytoplasmic domain into a T cell to make the T cell express a complex of NKp30 receptor and become a multi-chain CAR-T cell, the CAR-T cell can form stable immune synapses with cancer cells, thereby exerting excellent cytotoxicity to the cancer cells.

Preferably, the foresaid extracellular antigen binding domain comprises a heavy chain variable region of anti-mesothelin (MSLN) antibody, a heavy chain variable region of anti-CD22 antibody, a heavy chain variable region of anti-CD19 antibody, a heavy chain variable region of anti-BCMA (B-cell maturation antigen) antibody, a heavy chain variable region of anti-CD123 antibody, a heavy chain variable region of anti-GPRC5D (G protein-coupled receptor, class C, group 5, member D) antibody or a heavy chain variable region of anti-TSHR (thyroid-stimulating hormone receptor) antibody.

In one embodiment, the foresaid extracellular antigen binding domain may be selected from heavy chain variable regions derived from heavy chain antibodies (VHH) or a single-chain variable fragment (scFv); wherein, the said heavy chain variable regions derived from heavy chain antibodies may be derived from a single-chain binding region of heavy chain antibodies from Camelidae.

In one embodiment, the foresaid extracellular antigen binding domain may be a heavy chain variable region of anti-BCMA heavy chain antibody.

In one embodiment, the foresaid extracellular antigen binding domain may comprise a first heavy chain variable region of anti-BCMA heavy chain antibody and a second heavy chain variable region of anti-BCMA heavy chain antibody linked by G₄S peptide, which encodes the amino acid sequence of SEQ ID NO: 1. The first heavy chain variable region of anti-BCMA heavy chain antibody encodes the amino acid sequence of SEQ ID NO: 2 and the second heavy chain variable region of anti-BCMA heavy chain antibody encodes the amino acid sequence of SEQ ID NO: 3. The first heavy chain variable region of anti-BCMA heavy chain antibody comprises complementarity-determining regions (CDRs) shown as the following: CDRH1 encoding the amino acid sequence of SEQ ID NO: 4; CDRH2 encoding the amino acid sequence of SEQ ID NO: 5; and CDRH3 encoding the amino acid sequence of SEQ ID NO: 6. The second heavy chain variable region of anti-BCMA heavy chain antibody comprises CDRs shown as the following: CDRH1 encoding the amino acid sequence of SEQ ID NO: 7; CDRH2 encoding the amino acid sequence of SEQ ID NO: 8; and CDRH3 encoding the amino acid sequence of SEQ ID NO: 9.

In one embodiment, the foresaid extracellular antigen binding domain may comprise the heavy chain variable region of anti-CD 19 antibody. The heavy chain variable region of anti-CD19 antibody comprises CDRs shown as the following: CDRH1 encoding the amino acid sequence of SEQ ID NO: 10; CDRH2 encoding the amino acid sequence of SEQ ID NO: 11; and CDRH3 encoding the amino acid sequence of SEQ ID NO: 12. In one embodiment, the foresaid extracellular antigen binding domain may further comprise a light chain variable region of anti-CD 19 antibody, and the light chain variable region of anti-CD 19 antibody comprises CDRs shown as the following: CDRL1 encoding the amino acid sequence of SEQ ID NO: 13; CDRL2 encoding the amino acid sequence of SEQ ID NO: 14; and CDRL3 encoding the amino acid sequence of SEQ ID NO: 15. In one embodiment, the foresaid extracellular antigen binding domain may be FMC63 scFv encoding the amino acid sequence of SEQ ID NO: 16.

In one embodiment, the foresaid extracellular antigen binding domain may comprise the heavy chain variable region of anti-mesothelin antibody, and the heavy chain variable region of anti-mesothelin antibody comprises CDRs shown as the following: CDRH1 encoding the amino acid sequence of SEQ ID NO: 17; CDRH2 encoding the amino acid sequence of SEQ ID NO: 18; and CDRH3 encoding the amino acid sequence of SEQ ID NO: 19. In one embodiment, the foresaid extracellular antigen binding domain may further comprise a light chain variable region of anti-mesothelin antibody, and the light chain variable region of anti-mesothelin antibody comprises CDRs shown as the following: CDRL1 encoding the amino acid sequence of SEQ ID NO: 20; CDRL2 encoding the amino acid sequence of SEQ ID NO: 21; and CDRL3 encoding the amino acid sequence of SEQ ID NO: 22. In one embodiment, the foresaid extracellular antigen binding domain may be SS1 scFv encoding the amino acid sequence of SEQ ID NO: 23.

In one embodiment, the foresaid extracellular antigen binding domain may comprise the heavy chain variable region of anti-BCMA antibody, and the heavy chain variable region of anti-BCMA antibody comprises CDRs shown as the following: CDRH1 encoding the amino acid sequence of SEQ ID NO: 24; CDRH2 encoding the amino acid sequence of SEQ ID NO: 25; and CDRH3 encoding the amino acid sequence of SEQ ID NO: 26. In one embodiment, the foresaid extracellular antigen binding domain may further comprise a light chain variable region of anti-BCMA antibody, and the light chain variable region of anti-BCMA antibody comprises CDRs shown as the following: CDRL1 encoding the amino acid sequence of SEQ ID NO: 27; CDRL2 encoding the amino acid sequence of SEQ ID NO: 28; and CDRL3 encoding the amino acid sequence of SEQ ID NO: 29. In one embodiment, the foresaid extracellular antigen binding domain may be J6M0 scFv encoding the amino acid sequence of SEQ ID NO: 30. In another embodiment, the heavy chain variable region of anti-BCMA antibody comprises CDRs shown as the following: CDRH1 encoding the amino acid sequence of SEQ ID NO: 31; CDRH2 encoding the amino acid sequence of SEQ ID NO: 32; and CDRH3 encoding the amino acid sequence of SEQ ID NO: 33. In one embodiment, the foresaid extracellular antigen binding domain may further comprise a light chain variable region of anti-BCMA antibody, and the light chain variable region of anti-BCMA antibody comprises CDRs shown as the following: CDRL1 encoding the amino acid sequence of SEQ ID NO: 34; CDRL2 encoding the amino acid sequence of SEQ ID NO: 35; and CDRL3 encoding the amino acid sequence of SEQ ID NO: 36. In one embodiment, the foresaid extracellular antigen binding domain may be BB2121 scFv encoding the amino acid sequence of SEQ ID NO: 37.

In one embodiment, the foresaid extracellular antigen binding domain may comprise the heavy chain variable region of anti-CD22 antibody, and the heavy chain variable region of anti-CD22 antibody comprises CDRs shown as the following: CDRH1 encoding the amino acid sequence of SEQ ID NO: 38; CDRH2 encoding the amino acid sequence of SEQ ID NO: 39; and CDRH3 encoding the amino acid sequence of SEQ ID NO: 40. In one embodiment, the foresaid extracellular antigen binding domain may further comprise a light chain variable region of anti-CD22 antibody, and the light chain variable region of anti-CD22 antibody comprises CDRs shown as the following: CDRL1 encoding the amino acid sequence of SEQ ID NO: 41; CDRL2 encoding the amino acid sequence of SEQ ID NO: 42; and CDRL3 encoding the amino acid sequence of SEQ ID NO: 43. In one embodiment, the foresaid extracellular antigen binding domain may be m971 scFv encoding the amino acid sequence of SEQ ID NO: 44. In another embodiment, the foresaid extracellular antigen binding domain may comprise the heavy chain variable region of anti-CD22 antibody, and the heavy chain variable region of anti-CD22 antibody comprises CDRs shown as the following: CDRH1 encoding the amino acid sequence of SEQ ID NO: 45; CDRH2 encoding the amino acid sequence of SEQ ID NO: 46; and CDRH3 encoding the amino acid sequence of SEQ ID NO: 47. In one embodiment, the foresaid extracellular antigen binding domain may further comprise a light chain variable region of anti-CD22 antibody, and the light chain variable region of anti-CD22 antibody comprises CDRs shown as the following: CDRL1 encoding the amino acid sequence of SEQ ID NO: 48; CDRL2 encoding the amino acid sequence of SEQ ID NO: 49; and CDRL3 encoding the amino acid sequence of SEQ ID NO: 50. In one embodiment, the foresaid extracellular antigen binding domain may be LL2 scFv encoding the amino acid sequence of SEQ ID NO: 51. In another embodiment, the foresaid extracellular antigen binding domain may comprise the heavy chain variable region of anti-CD22 antibody, and the heavy chain variable region of anti-CD22 antibody comprises CDRs shown as the following: CDRH1 encoding the amino acid sequence of SEQ ID NO: 52; CDRH2 encoding the amino acid sequence of SEQ ID NO: 53; and CDRH3 encoding the amino acid sequence of SEQ ID NO: 54. In one embodiment, the foresaid extracellular antigen binding domain may further comprise a light chain variable region of anti-CD22 antibody, and the light chain variable region of anti-CD22 antibody comprises CDRs shown as the following: CDRL1 encoding the amino acid sequence of SEQ ID NO: 55; CDRL2 encoding the amino acid sequence of SEQ ID NO: 56; and CDRL3 encoding the amino acid sequence of SEQ ID NO: 57. In one embodiment, the foresaid extracellular antigen binding domain may be G5/44 scFv encoding the amino acid sequence of SEQ ID NO: 58. In another embodiment, the foresaid extracellular antigen binding domain may comprise the heavy chain variable region of anti-CD22 antibody, and the heavy chain variable region of anti-CD22 antibody comprises CDRs shown as the following: CDRH1 encoding the amino acid sequence of SEQ ID NO: 59; CDRH2 encoding the amino acid sequence of SEQ ID NO: 60; and CDRH3 encoding the amino acid sequence of SEQ ID NO: 61. In one embodiment, the foresaid extracellular antigen binding domain may further comprise a light chain variable region of anti-CD22 antibody, and the light chain variable region of anti-CD22 antibody comprises CDRs shown as the following: CDRL1 encoding the amino acid sequence of SEQ ID NO: 62; CDRL2 encoding the amino acid sequence of SEQ ID NO: 63; and CDRL3 encoding the amino acid sequence of SEQ ID NO: 64. In one embodiment, the foresaid extracellular antigen binding domain may be RFB4 scFv encoding the amino acid sequence of SEQ ID NO: 65.

Preferably, the foresaid extracellular antigen binding domain may comprise the heavy chain variable region of anti-CD19 antibody, and the foresaid extracellular antigen binding domain may be FMC63 scFv.

Preferably, the foresaid extracellular antigen binding domain may comprise the heavy chain variable region of anti-mesothelin antibody, and the foresaid extracellular antigen binding domain may be SS1 scFv.

Preferably, the foresaid extracellular antigen binding domain may comprise the heavy chain variable region of anti-BCMA antibody, and the foresaid extracellular antigen binding domain may be J6M0 scFv or BB2121 scFv.

Preferably, the foresaid extracellular antigen binding domain may comprise the heavy chain variable region of anti-CD22 antibody, and the foresaid extracellular antigen binding domain may be m971 scFv, LL2 scFv, G5/44 scFv or RFB4 scFv.

Preferably, the foresaid hinge domain may be selected from the group consisting of: (G₄S)₃ (shown as SEQ ID NO: 66), a hinge domain of CD8 (shown as SEQ ID NO: 67), a hinge domain of CD28 (shown as SEQ ID NO: 68), a hinge domain of IgG4 (shown as SEQ ID NO: 69), EAAAKGGGGS (shown as SEQ ID NO: 70), (EAAAK)₃ (shown as SEQ ID NO: 71), a hinge domain of GST (shown as SEQ ID NO: 72), a hinge domain of EAAAK-GS (shown as SEQ ID NO: 73), a hinge domain of IgD (shown as SEQ ID NO: 74), an IgG4-CH3 domain (shown as SEQ ID NO: 75) and (AP)₆ (shown as SEQ ID NO: 76).

Preferably, the foresaid isolated nucleic acid molecule may further comprise a nucleic acid sequence encoding an adapter. More preferably, the said adapter may be CD3ζ, DAP10, CD79, FcεR1γ, FcRy or Fcβ/γ. The present disclosure adopts the full-length protein of CD3ζ, DAP10, CD79, FcεR1γ, FcRy or Fcβ/γ as the adapter.

Preferably, the foresaid isolated nucleic acid molecule may further comprise a nucleic acid sequence encoding signal transduction domain of CD3ζ, a nucleic acid sequence encoding FcεR1γ adapter or a nucleic acid sequence encoding T2A self-cleaving peptide. For example, the nucleic acid sequence encoding FcεR1γ adapter may be located at 5' end of the foresaid isolated nucleic acid molecule; and the nucleic acid sequence encoding T2A self-cleaving peptide may be located between the nucleic acid sequence encoding FcεR1γ adapter and the nucleic acid sequence encoding the extracellular antigen binding domain.

Preferably, the hinge domain and the NKp30 transmembrane domain are linked by a NKp30 stalk domain.

Preferably, in the foresaid isolated nucleic acid molecule, when the extracellular antigen binding domain comprises a heavy chain variable region of anti-CD19 antibody and the hinge domain is (EAAAK)₃, this specific combination of the extracellular antigen binding domain and the hinge domain can result in lentivirus with good transduction efficiency. CAR-Ts (CAR-T cells) produced with this specific combination expand at greater rates, possess higher proportions of stem cell-like memory T-cells (Tscm cells) and exhibit stronger cytotoxicity. More preferably, the foresaid heavy chain variable region of anti-CD19 antibody may be a fragment of FMC63 scFv.

Preferably, in the foresaid isolated nucleic acid molecule, when the extracellular antigen binding domain comprises a heavy chain variable region of anti-mesothelin antibody and the hinge domain is (G₄S)₃, this specific combination of the extracellular antigen binding domain and the hinge domain can result in lentivirus with good transduction efficiency. CAR-Ts produced with this specific combination expand at greater rates, possess higher proportions of Tscm cells and exhibit stronger cytotoxicity. More preferably, the foresaid heavy chain variable region of anti-mesothelin antibody may be a fragment of SS1 scFv.

Preferably, in the foresaid isolated nucleic acid molecule, when the extracellular antigen binding domain comprises a heavy chain variable region of anti-BCMA antibody and the hinge domain is the hinge of CD28, this specific combination of the extracellular antigen binding domain and the hinge domain can result in lentivirus with good transduction efficiency. CAR-Ts produced with this specific combination expand at greater rates, possess higher proportions of Tscm cells and exhibit stronger cytotoxicity. More preferably, the foresaid heavy chain variable region of anti-BCMA antibody may be a fragment of J6M0 scFv.

To achieve the foresaid objective, the present disclosure further provides a plasmid, which comprises the foresaid isolated nucleic acid molecule, and the plasmid may be a DNA plasmid or an RNA plasmid. The sequential order of nucleic acid fragments from 5' end to 3' end of the plasmid may be FcεR1γ adapter, T2A self-cleaving peptide, the extracellular antigen binding domain, the hinge domain, the NKp30 transmembrane domain and then the NKp30 cytoplasmic domain. In one embodiment, the sequential order of nucleic acid fragments from 5' end to 3' end of the plasmid may be 5' LTR, promoter, FcεR1γ adapter, T2A self-cleaving peptide, the extracellular antigen binding domain, the hinge domain, the NKp30 transmembrane domain, the NKp30 cytoplasmic domain and 3' LTR.

To achieve the foresaid objective, the present disclosure further provides a method for preparing a chimeric antigen receptor cell, specifically a chimeric antigen receptor gene-modified cell, which comprises introducing the foresaid isolated nucleic acid molecule into a nucleated cell.

Preferably, in the foresaid method for preparing a chimeric antigen receptor cell, the said "introducing" is achieved by lentivirus transduction.

Preferably, the foresaid nucleated cell may be leukocytes.

Preferably, the foresaid nucleated cell may be T cells.

To achieve the foresaid objective, the present disclosure further provides an isolated cell, which comprises the foresaid isolated nucleic acid molecule.

Preferably, the foresaid isolated cell may comprise a T cell, a natural killer cell, a natural killer T cell or an adipose-derived stem cell (ADSC).

To achieve the foresaid objective, the present disclosure further provides a use of the foresaid isolated cell to prepare a medication for treating or alleviating cancer, which means that the present disclosure further provides a pharmaceutical composition for treating or alleviating a cancer, comprising an effective amount of the foresaid isolated cell. For example, the foresaid cancer may be, but is not limited to, B-cell acute lymphoblastic leukemia, B-cell lymphoma, Leydig cell tumor, ovarian cancer, acute myeloid leukemia, mesothelioma, colon cancer, lung cancer, pancreatic cancer, thyroid cancer and/or multiple myeloma. Specifically, the use indicates a method of utilizing the foresaid isolated cell to prepare a medication for treating or alleviating cancer.

To achieve the foresaid objective, the present disclosure further provides a method for treating or alleviating cancer, which comprises administering to a subject in need thereof a medication comprising an effective amount of the foresaid isolated cell.

To achieve the foresaid objective, the present disclosure further provides a use of the foresaid isolated cell to prepare a medication for treating or alleviating an autoimmune disease, which means that the present disclosure further provides a pharmaceutical composition for treating or alleviating an autoimmune disease, comprising an effective amount of the foresaid isolated cell; wherein, the said autoimmune disease may be a disease related to autoimmune reaction that can be treated by CD19 CAR-T cell. In one embodiment, the foresaid autoimmune disease may be, but is not limited to, systemic lupus erythematosus (SLE), idiopathic inflammatory myositis, systemic sclerosis or multiple sclerosis. Specifically, the use indicates a method of utilizing the foresaid isolated cell to prepare a medication for treating or alleviating an autoimmune disease.

To achieve the foresaid objective, the present disclosure further provides a method for treating or alleviating an autoimmune disease, which comprises administering to a subject in need thereof a medication comprising an effective amount of the foresaid isolated cell.

The advantages of the present disclosure are described as the following: the present disclosure introduces nucleic acid sequences of the NKp30 transmembrane domain and the NKp30 cytoplasmic domain into a T cell to make the T cell express a complex that includes components of a of NKp30 receptor and become a multi-chain CAR-T cell. Such CAR-T cell is expected to form stable immune synapses with cancer cells, thereby enabling them to effectively kill cancer cells. Not only do these CAR-T cells significantly reduce the number of existing viable cancer cells, their anti-tumor effects are persistent in preventing recurrent cancer cell or tumor growth; meanwhile, the resulting CAR-T cells also possess high proportions of Tscm cells, and it is expected to display reduced cytokine release syndrome and neurotoxicity caused by such therapy.

Other objectives, advantages, and novel features of the disclosure will become more apparent in the following detailed descriptions when presented in conjunction with their respective drawings.

In the drawings:
Fig. 1 shows a schematic illustration of the pLAS5w vector;
Fig. 2 shows a schematic illustration of plasmids for Examples 1-1A to 1-5A and Comparative Example 1A;
Fig. 3 shows a schematic illustration of cell membrane protein structures for CAR-T cells of Examples 1-1C to 1-5C and Comparative Example 1C;
Figs. 4A and 4B respectively show anti-CD19 receptor and NKp30 receptor expressions of CAR-Ts of Example 1-4C determined by flow cytometric analyses in Test Example 3;
Figs. 4C and 4D respectively show anti-CD19 receptor and NKp30 receptor expressions of CAR-Ts of Example 1-5C determined by flow cytometric analyses in Test Example 3;
Figs. 4E and 4F respectively show anti-CD19 receptor and NKp30 receptor expressions of CAR-Ts of Comparative Example 1C determined by flow cytometric analyses in Test Example 3;
Figs. 5A, 5B and 5C respectively show the distribution of T cell subpopulations of CAR-Ts of Example 1-5C, Comparative Example 1C and the un-transduced group analyzed by flow cytometry in Test Example 4;
Fig. 6 shows the cytotoxicity percentage against luciferase-expressing Raji cells of CAR-T cells of Example 1-5C and Comparative Example 1C, and T cells of the un-transduced group at an E:T ratio of 3:1 in Test Example 5, determined using a luciferase killing assay;
Fig. 7A shows the real-time changes in normalized cell index signals that represent the number of viable, truncated CD19-expressing cancer cells, as a response to CAR-T cells of Example 1-5C and Comparative Example 1C, and T cells of the un-transduced group at an E:T ratio of 0.1 to 1 in Test Example 6, determined using a real-time cytotoxicity assay;
Fig. 7B shows the cytotoxicity percentage against truncated CD19-expressing SKOV3 cells of CAR-T cells of Example 1-5C and Comparative Example 1C, and T cells of the un-transduced group at an E:T ratio of 0.1:1 and at 72 hours post-treatment in Test Example 6;
Fig. 8 shows a schematic illustration of plasmids for Example 2A and Comparative Example 2A;
Figs. 9A and 9B respectively show schematic illustrations of cell membrane protein structures for CAR-T cells of Example 2C and Comparative Example 2C;
Figs. 10A and 10B respectively show anti-mesothelin receptor and NKp30 receptor expressions of CAR-Ts of Example 2C determined by flow cytometric analyses in Test Example 8;
Figs. 10C and 10D respectively show anti-mesothelin receptor and NKp30 receptor expressions of CAR-Ts of Comparative Example 2C determined by cytometric analyses in Test Example 8;
Figs. 11A, 11B and 11C respectively show the distribution of T cell subpopulations of CAR-Ts of Example 2C, Comparative Example 2C and the un-transduced group analyzed by flow cytometry in Test Example 10;
Figs. 12A, 12B and 12C respectively show, at E:T ratios of 3:1, 1:1 and 0.3:1, the real-time changes in normalized cell index signals that represent the number of viable Hela cells, as a response to CAR-T cells of Example 2C and Comparative Example 2C, and T cells of the un-transduced group in Test Example 11;
Figs. 13A, 13B and 13C respectively show original, 1^{st} repeat, and 2^{nd} repeat, administered once daily, of repeated challenges of fresh, viable Hela cancer cells toward CAR-T cells of Example 2C and Comparative Example 2C, and T cells of the un-transduced group at an E:T ratio of 1:1 in Test Example 12,; wherein, cytotoxicity of CAR-Ts are indicated by real-time changes in the normalized cell index signals which represent the number of viable Hela cells;
Fig. 14 shows a schematic illustration of plasmids for Example 3A and Comparative Example 3A;
Fig. 15 shows a schematic illustration of cell membrane protein structures for CAR-T cells of Example 3C and Comparative Example 3C;
Figs. 16A and 16B respectively show anti-BCMA receptor and NKp30 receptor expressions of CAR-Ts of Example 3C determined by flow cytometric analyses in Test Example 15;
Fig. 16C shows anti-BCMA receptor expression of CAR-Ts of Comparative Example 3C determined by flow cytometric analyses in Test Example 15;
Figs. 17A, 17B and 17C respectively show the distribution of T cell subpopulations of CAR-Ts of Example 3C, Comparative Example 3C and the un-transduced group analyzed by flow cytometry in Test Example 16;
Fig. 18 shows the cytotoxicity percentage against BCMA-expressing SKOV3 cancer cells of CAR-Ts of Example 3C, Comparative Example 3C and the un-transduced group analyzed by flow cytometry in Test Example 17;
Figs. 19A and 19B respectively show, at E:T ratios of 0.3:1 and 0.1:1, the real-time changes in normalized cell index signals that represent the number of viable BCMA-expressing SKOV3 cancer cells, as a response to CAR-T cells of Example 3C and Comparative Example 3C, and T cells of the un-transduced group in Test Example 18.

Hereinafter, some embodiments and test examples are exemplified to illustrate the implementation and the effects of the present disclosure. One person skilled in the art can easily realize the advantages and effects of the present disclosure in accordance with the contents of the specification.

### I. Production and characterization of CAR-T expressing anti-CD19-NKp30

### Examples 1-1 to 1-5: Anti-CD19-NKp30 nucleic acid molecule design

First, a nucleic acid sequence encoding full length protein of FcεR1γ adapter (shown as SEQ ID NO: 77) and a nucleic acid sequence encoding FMC63 scFv capable of targeting CD19 (shown as SEQ ID NO: 78) were provided. After the foresaid two nucleic acid sequences were linked to obtain a linked sequence, the 3' end of the linked sequence was linked to the 5' end of a nucleic acid sequence of NKp30 without immunoglobulin-like domain (shown as SEQ ID NO: 79), wherein the nucleic acid sequence of NKp30 without immunoglobulin-like domain comprised NKp30 stalk domain, NKp30 transmembrane domain and NKp30 cytoplasmic domain. With this method, anti-CD19-NKp30 nucleic acid molecules of Examples 1-1 to 1-5 were obtained. More specifically, the nucleic acid sequences of FcεR1γ and FMC63 were linked by a nucleic acid sequence encoding T2A self-cleaving peptide (shown as SEQ ID NO: 80), and the nucleic acid sequence of NKp30 without immunoglobulin-like domain and the nucleic acid sequence of FMC63 could be linked by nucleic acid sequences of different hinge domains. The nucleic acid sequences of hinge domains used by Examples 1-1 to 1-5 respectively were (G₄S)₃ (shown as SEQ ID NO: 66), a hinge domain of CD28 (shown as SEQ ID NO: 68), a hinge domain of CD8 (shown as SEQ ID NO: 67), a hinge domain of IgG4 (shown as SEQ ID NO: 69) and (EAAAK)₃ (shown as SEQ ID NO: 71); wherein, the hinge domain of CD28, the hinge domain of CD8 and the hinge domain of IgG4 were derived from human. The nucleic acid sequences of the nucleic acid molecules of Examples 1-1 to 1-5 are shown as SEQ ID NO: 81 to 85, respectively.

The preparation of Comparative Example 1, which did not have the above-mentioned NKp30 without immunoglobulin-like domain, was based on the following approach: the 3' end of the nucleic acid sequence encoding FMC63 was linked to a nucleic acid sequence encoding transmembrane domain of CD8 (shown as SEQ ID NO: 86), and the FMC63 and the transmembrane domain of CD8 were linked by a hinge domain of CD8. Then, the 3' end of the nucleic acid sequence encoding transmembrane domain of CD8 was further linked to a nucleic acid sequence encoding 4-1BB (shown as SEQ ID NO: 87) and a nucleic acid sequence encoding signal transduction domain of CD3ζ (shown as SEQ ID NO: 88). The nucleic acid sequence of the nucleic acid molecule of Comparative Examples 1 is shown as SEQ ID NO: 89.

The hinge domains of Examples 1-1 to 1-5 (abbreviated as E1-1 to E1-5) and Comparative Example 1 (abbreviated as CE1) are also shown in Table 1.

**Table 1: the hinge domains of Examples 1-1 to 1-5 and Comparative Example 1**

| Group | E1-1 | E1-2 | E1-3 | E1-4 | E1-5 | CE1 |
|---|---|---|---|---|---|---|
| Hinge domain | (G₄S)₃ | Hinge domain of CD28 | Hinge domain of CD8 | Hinge domain of IgG4 | (EAAAK)₃ | Hinge domain of CD8 |

### Examples 1-1A to 1-5A: Construction of anti-CD19-NKp30 plasmids

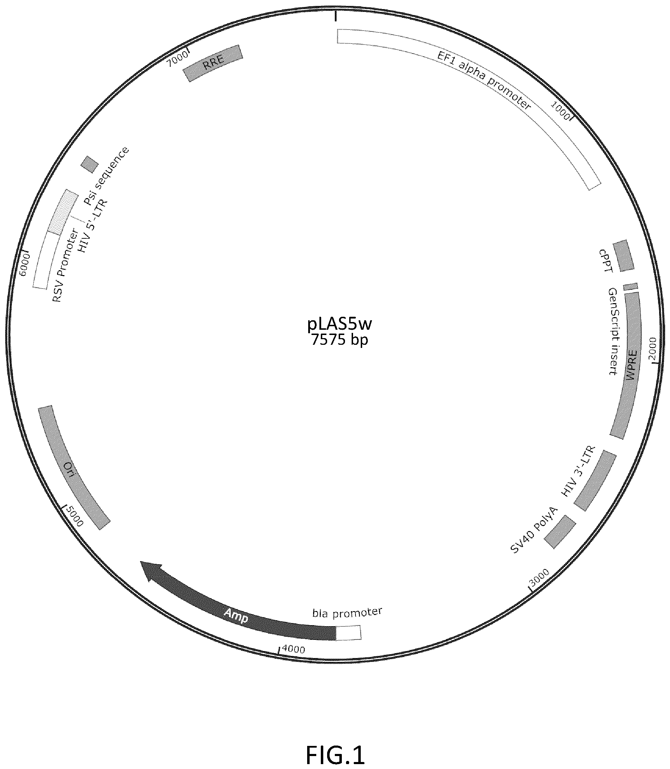

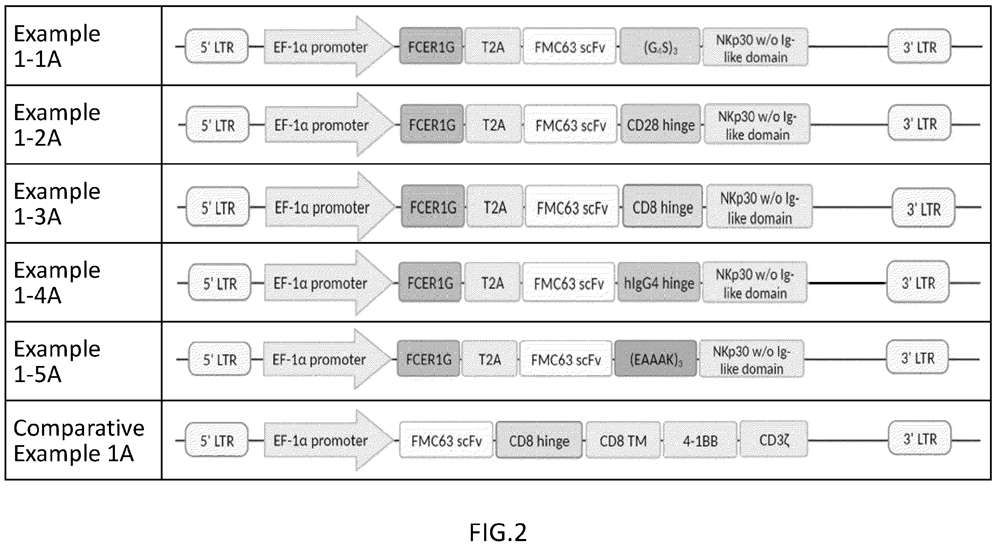

As shown in Fig. 1, the pLAS5w.Ppuro vector comprising a nucleotide sequence of TAR-reserved chimeric 5' LTR and driven by RSV promoter was purchased from Academia Sinica RNAi Core facility, Product No.: C6-8-39. After hPGK promoter and PAC gene of the pLAS5w.Ppuro vector were deleted (conducted by GenScript), a pLAS5w vector was obtained. The nucleic acid molecules of Examples 1-1 to 1-5 and Comparative Example 1 were respectively ligated into the pLAS5w vector by using restriction enzymes of HpaI and EcoRI, and anti-CD19-NKp30 plasmids of Examples 1-1A to 1-5A and a plasmid of Comparative Example 1A were obtained and are shown in Fig. 2.

### Examples 1-1B to 1-5B: Production of anti-CD19-NKp30 lentiviruses

Lentivirus particles of Examples 1-1B to 1-5B and Comparative Example 1B were produced by transfecting human embryonic kidney cells 293 (HEK 293 cells, ATCC, CRL-3216) with target genes using Polyjet transfection reagent (purchased from SignaGen Laboratories, SL100688). Specifically, 50 µg of plasmids of Examples 1-1A to 1-5A or Comparative Example 1A comprising the target genes were adopted as the transfer plasmid; 45 µg of pCMV deltaR8.91 (purchased from Academia Sinica) was adopted as the packaging plasmid; and 5 µg of pMD.G (purchased from Academia Sinica) was adopted as the envelope plasmid. Then, the foresaid transfer plasmid, packaging plasmid and envelope plasmid were incubated with 300 µL of Polyjet transfection reagent in 10 mL of serum-free DMEM (Dulbecco's Modified Eagle Medium) for 15 minutes to obtain a Polyjet/DNA mixture. Then, the Polyjet/DNA mixture was added into 100 mL of DMEM with 10% FBS (fetal bovine serum), and then added dropwise into a T875 flask that was previously seeded with 9×10⁷ HEK293 cells. After reacting for 12 to 18 hours transfection (such as transfecting for 16 hours), the medium was replaced with 100 mL of Opti-MEM (purchased from Gibco, 31985070) containing 1 mM of sodium pyruvate (purchased from Sartorius). After transfecting for another 48 hours, the supernatant containing lentivirus particles was collected as the first batch of lentivirus. The transfection culture medium was then replenished with fresh Opti-MEM containing 1 mM of sodium pyruvate. After another 24 hours, the supernatant containing lentivirus particles was collected again as the second batch of lentivirus. The first and second batches of lentivirus-containing supernatants were independently centrifuged at 1500 rpm, 4°C for 10 minutes to remove cell debris, respectively. Then, the supernatants were filtered by a filter of 0.45 µm pore size (purchased from Membrane-solutions), respectively and then combined to obtain a filtered supernatant. Afterward, the filtered supernatant was concentrated by Lenti-X reagent (purchased from Takara Bio) at a ratio of 3:1, *i.e.,* the volume ratio of the filtered supernatant and Lenti-X reagent was 3:1. Specifically, the filtered supernatant and Lenti-X reagent were mixed by rotating it upside down at 4°C overnight, and then the mixture was centrifuged at 1500 rpm, 4°C for 45 minutes. After the supernatant was carefully removed, the pellet was resuspended in 2 mL of X-VIVO^{™} 15 medium (purchased from Lonza) to obtain concentrated lentiviruses of Examples 1-1B to 1-5B and concentrated lentivirus of Comparative Example 1B, which were typically stored at -80°C until use.

### Test Example 1: Titer determination of anti-CD19-NKp30 lentiviruses of Examples 1-1B to 1-5B

Viral titers were determined by transducing 4×10⁴ Jurkat cells (T cells of human acute leukemia) using lentivirus samples that were 3-fold serial diluted, wherein, the lentivirus samples used in Test Example 1 were the concentrated lentiviruses of Examples 1-1B to 1-5B and the concentrated lentivirus of Comparative Example 1B. Specifically, to dilute each concentration of lentivirus by 3-fold, 50 µL of each foresaid lentivirus sample was added into 100 µL of X-VIVO^{™} 15 medium, and repeated further with the addition of X-VIVO^{™} 15 medium, until the final dilution of the lowest lentiviral titering standard reached 6561-fold dilution of the original concentrated lentivirus, thereby obtaining serval groups of reconstituted, diluted lentivirus samples. Next, 50 µL of each reconstituted, diluted lentivirus sample was added into a 96-well round-bottom plate, to be cultured with 4×10⁴ Jurkat cells, at a cell density of 4×10⁵ cells/mL. The lentiviral transduction was performed for 72 hours. To detect Jurkat cells that were successfully transduced, 200 ng of Biotinylated Human CD19, Fc tag (purchased from ACROBiosystems, CD9-H8259) was adopted for targeting anti-CD19 receptor, and 500-fold diluted Streptavidin conjugated phycoerythrin (PE) (purchased from Invitrogen, 12-4317-87) was adopted for detecting the expression of the anti-CD19 receptor. A flow cytometer (purchased from Sony, SA3800) was used for analyzing the expression of the target protein, thereby determining the transduction efficiency of lentivirus. Un-transduced Jurkat cells were used as the negative control. The functional viral titer of lentivirus with anti-CD19-NKp30 was calculated by the following equation, and the experimental results are shown in Table 2. The resulting functional viral titers were then used to determine the appropriate amount of lentivirus to be applied for CAR-T cell production.

Functional Titer of Lentivirus [transducing unit (TU) / mL] = (percentage of cells expressing anti-CD19 receptor) × 4×10⁴ (cells) × 20 × dilution Fold.

**Table 2: the functional viral titer of lentiviruses of Examples 1-1B to 1-5B (abbreviated as E1-1B to E1-5B) and Comparative Example 1B (abbreviated as CE1B)**

| Group | E1-1B | E1-2B | E1-3B | E1-4B | E1-5B | CE1B |
|---|---|---|---|---|---|---|
| Functional viral titer (TU/mL) | 1×10⁶ | about 1×10⁵ | 3.29×10⁵ | about 2×10⁶ to 6.8×10⁶ | 2.14×10⁶ | 5.7×10⁷ |

When the functional viral titer was more than or equal to 1×10⁶ TU/mL, T cells could be successfully transduced and become CAR-T cells with a lower amount of lentivirus, whereas, a lower volume of lentivirus used during transduction also tended to correlate with better growth of T cells for CAR-T production. Therefore, according to the results in Table 2, Example 1-1B, Example 1-4B, Example 1-5B and Comparative Example 1B met the foresaid criteria, which further benefited preparation of CAR-T cells. Moreover, Table 2 demonstrated that the functional viral titer of Example 1-4B, which had the hinge domain of IgG4, was the closest to the functional viral titer of Comparative Example 1B.

### Examples 1-1C, 1-4C and 1-5C: Manufacturing CAR-T cells that express anti-CD19-NKp30

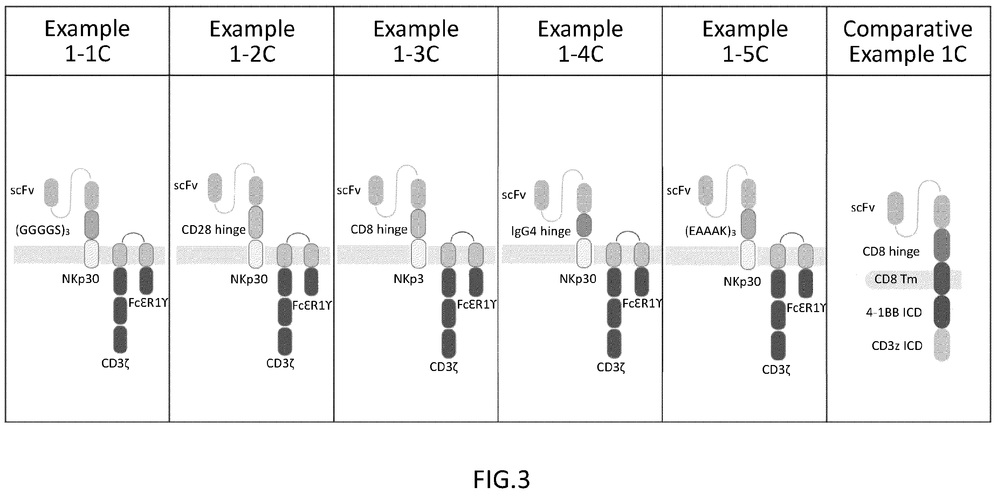

Fresh peripheral blood samples were collected from healthy volunteers under their consent. On day 0, human peripheral blood mononuclear cells (PBMCs) were isolated using SepMate centrifuge tubes (purchased from STEMCELL, 86450). In brief, the fresh blood samples were diluted with the same volume of PBS buffer containing 2% FBS to obtain diluted blood samples. Next, the diluted blood samples were separately added dropwise along tube wall of the SepMate centrifuge tube, during which the SepMate centrifuge tube remained in a vertical position so that the diluted blood sample and Ficoll density-gradient centrifugation solution could mix well in the SepMate centrifuge tube; and the SepMate centrifuge tubes containing these mixtures were then centrifuged at 1200×g at room temperature for 20 minutes. Next, the supernatant (rich in mononuclear cells) was decanted into a new tube for collection and repeatedly washed by PBS buffer containing 2% FBS, followed by centrifugation at 300×g at room temperature for 8 minutes to ultimately collect the pelleted PBMC cells kept through each wash. Next, Dynabeads coated with anti-CD3/CD28 antibodies (*i.e*., CTS (Cell Therapy Systems) Dynabeads CD3/CD28 (purchased from Gibco, Cat. No. 40203D); hereinafter referred to as CD3/CD28 Dynabeads) in combination with DynaMag-Spin (purchased from Thermo) were used to isolate T cells from the collected PBMCs. The proportion of CD3⁺ T cells in the isolated PBMCs was then measured by anti-CD3 antibody (purchased from Biolegend, Cat.No.300441) detection and flow cytometric analysis, and by also performing cell count for the total number of PBMCs, the quantity of the CD3⁺ T cells was calculated. Next, CD3/CD28 Dynabeads were mixed with PBMCs at a bead to cell ratio of 3 to 1 (*i.e.,* three times of beads than the CD3⁺ T cells). The CD3/CD28 Dynabeads and PBMC mixture was then incubated at room temperature for 30 to 60 minutes to allow the CD3/CD28 Dynabeads to bind to T cells. Next, DynaMag-Spin was used to select for CD3-positive T cells (CD3⁺ T cells) with the removal of B cells, NK cells and mononuclear cells unbound to the CD3 antibody at the same time; and complexes of CD3/CD28 Dynabeads and T cells formed by antigen-antibody binding were thereby obtained. The complexes of CD3/CD28 Dynabeads and T cells with a density of 2×10⁵ cells/mL were then dispersed into X-VIVO^{™} 15 medium (purchased from Lonza) containing 200 IU/mL of recombinant human IL-2 (purchased from R&D Systems), and then cultured at 37°C in 5% CO₂ for 24 hours to activate the T cells. On the next day, lentiviruses of Examples 1-1B, 1-4B and 1-5B and Comparative Example 1B were respectively added to the activated T cells, at a multiplicity of infection (MOI) of 2 and left for 48 hours for lentiviral transduction; thereby obtaining complexes of CD3/CD28 Dynabeads and CAR-T cells of Examples 1-1C, 1-4C and 1-5C and Comparative Example 1C. The T cells that were not transduced using lentivirus were referred to as the un-transduced group. Herein, the foresaid MOI = [viral titer (TU/mL) × viral volume (mL)] / total number of T cells. On day 3, following detachment of CD3/CD28 Dynabeads from the complexes of CD3/CD28 Dynabeads and CAR-T cells, CAR-T cells with anti-CD19-NKp30 of Examples 1-1C, 1-4C and 1-5C, and CAR-T cells of Comparative Example 1C were obtained, and the structural schematic illustration of each group is shown in Fig. 3; wherein, because the transduced nucleic acid sequences of Examples 1-1C, 1-4C and 1-5C comprised FcεR1γ, CAR-T cells of Examples 1-1C, 1-4C and 1-5C were named as multi-chain CAR-T cells; and because the transduced nucleic acid sequences of Comparative Example 1C did not comprise FcεR1γ, CAR-T cells of Comparative Example 1C was named as single-chain CAR-T cells. The foresaid resulting CAR-T cells were transferred to a new flask having X-VIVO^{™} 15 medium containing 200 IU/mL of recombinant human IL-2, and then cultured with the same conditions used in the foresaid viral transduction process for cell expansion. After CAR-T cells were expanded for 7 days in total (another 4 days following CD3/CD28 Dynabeads debeading), the cell number of the un-transduced group and CAR-T cells with anti-CD19-NKp30 were measured. During the day 4 to day 7 of culture period of CAR-T cells, additional medium was added every 1 to 3 days for the purpose of maintaining the cell density at around 1×10⁶ cells/mL.

### Test Example 2: Cell expansion rate of CAR-T cells of Examples 1-1C, 1-4C and 1-5C, and Comparative Example 1C

The cell expansion rates of CAR-T cells of Examples 1-1C, 1-4C and 1-5C were obtained by calculating the total cell number at a specific timepoint during the preparation of CAR-T cells with anti-CD19-NKp30 of Examples 1-1C, 1-4C and 1-5C. Specifically, on day 3 during the preparation of CAR-T cells, CAR-T cells with anti-CD19-NKp30 were quantified by staining cells with Acridine Orange/ Propidium Iodide (AO/PI) after CD3/CD28 Dynabeads were removed and then seeded in 12-well plates at a cell density of 1×10⁶ cells/mL. Cell viability, number, and density of CAR-Ts were automatically determined by an automated cell counter (Luna Automated Cell Counter, purchased from Logos Biosystems). CAR-T cells of known quantities were then subcultured and seeded in cell culture plates at cell densities between 2×10⁵ cells/mL and 5×10⁵ cells/mL for cell expansion. CAR-T cell numbers were measured using such method both on day 0 and day 7 to calculate for cell expansion rates, *i.e.,* fold of cell expansion, shown in Table 3; wherein, the cell expansion rate of CAR-T cells with anti-CD19-NKp30 was calculated by dividing the cell number measured on day 7 by that on day 0.

**Table 3: folds of cell expansion for CAR-T cells of Examples 1-1C, 1-4C and 1-5C (abbreviated as E1-1C, E1-4C and E1-5C), and Comparative Example 1C (abbreviated as CE1C)**

| Group | E1-1C | E 1-4C | E1-5C | CE1C |
|---|---|---|---|---|
| Fold of cell expansion (day 7 relative to day 0) | 27-fold | 10-fold | 9.48-fold | 14.17-fold |

When the cell expansion rate was greater than 5-fold (between day 0 and day 7), it was beneficial to the subsequent functional tests of T cells. Therefore, according to the results in Table 3, the folds of cell expansion for CAR-T cells of Example 1-1C, Example 1-4C and Example 1-5C met such criteria; wherein, CAR-T cells of Example 1-1C having (G₄S)₃ as hinge domain had the greatest cell expansion rate, which was significantly greater than CAR-T cells of Comparative Example 1C.

### Test Example 3: Resulting CAR-positive expression of CAR-T cells of Examples 1-1C, 1-4C and 1-5C, and Comparative Example 1C, indicating transduction efficiencies of lentiviruses of Examples 1-1B, 1-4B and 1-5B, and Comparative Example 1B

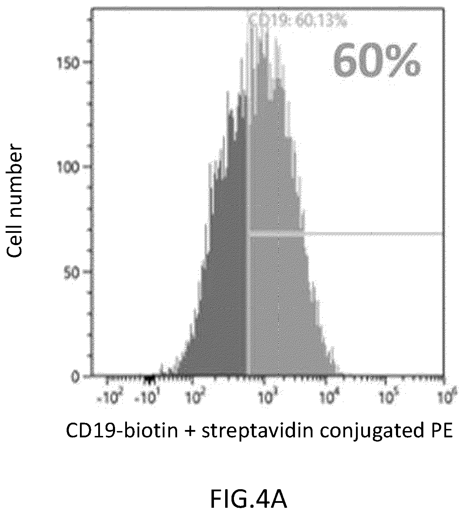

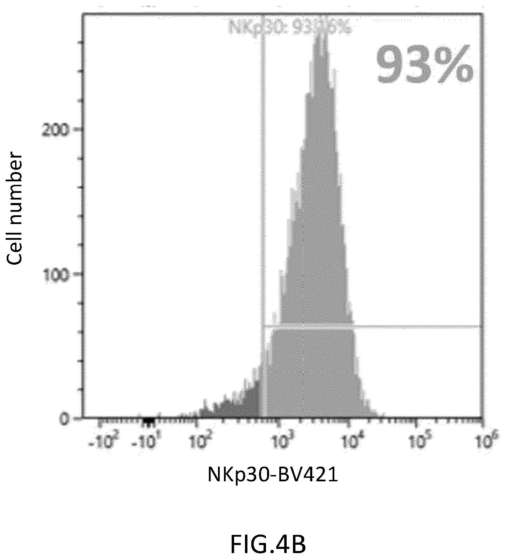

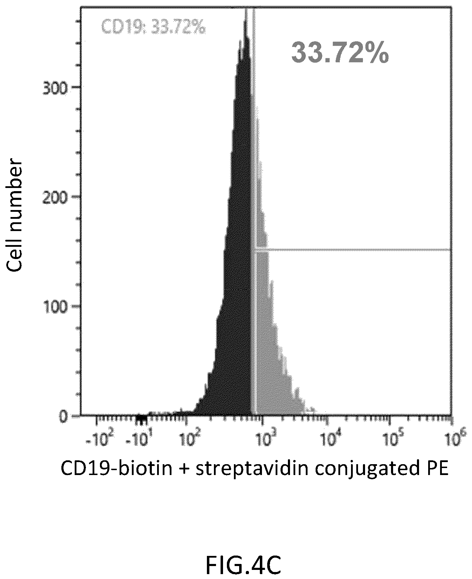

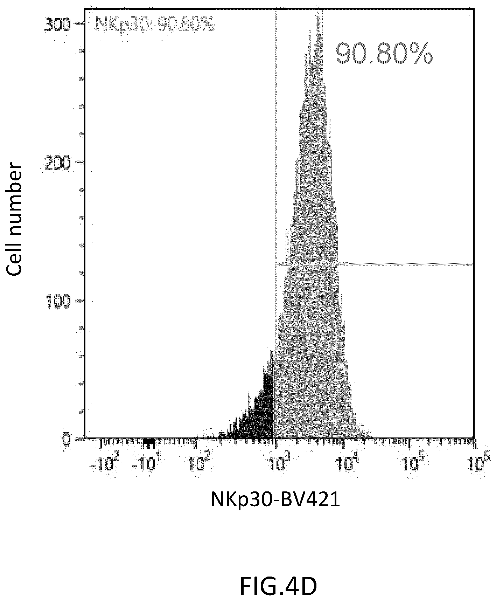

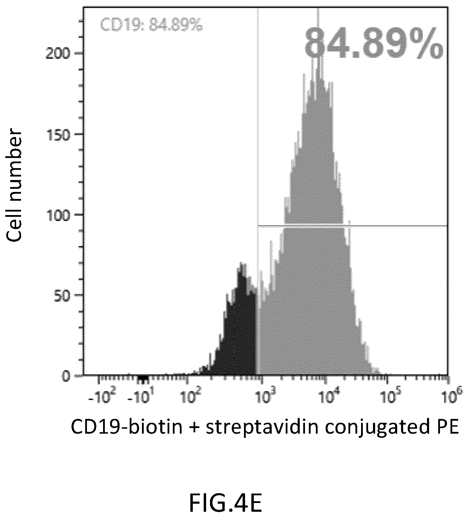

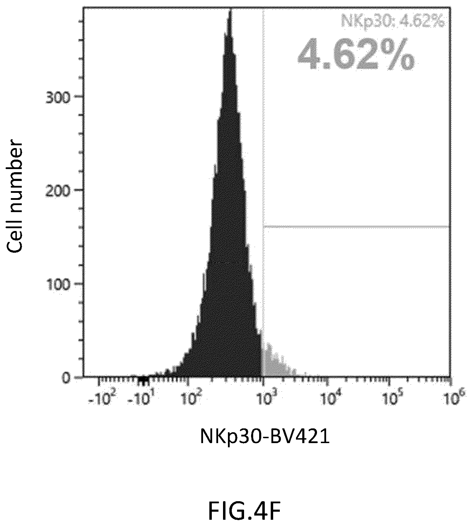

The expressions of anti-CD19 receptors of CAR-T cells of Examples 1-1C, 1-4C and 1-5C, and Comparative Example 1C were detected by using 100 µg/mL of Biotinylated Human CD19, Fc tag (purchased from ACROBiosystems, CD9-H8259) and 500-fold diluted Streptavidin conjugated phycoerythrin (PE) (purchased from Invitrogen, 12-4317-87) reconstituted in ice-cold PBS containing 2% FBS according to the operation manuals of manufacturers; while, the expressions of NKp30 were detected by adding 5 µL of anti-NKp30 antibody (purchased from BioLegend) conjugated with BV421 fluorescent dye reconstituted in the same buffer. Flow cytometric analyses of anti-CD19 receptor and NKp30 receptor expressions for CAR-T cells of Example 1-4C are shown in Fig. 4A and Fig. 4B, respectively; flow cytometric analyses of anti-CD19 receptor and NKp30 receptor expressions for CAR-T cells of Example 1-5C are shown in Fig. 4C and Fig. 4D, respectively; and flow cytometric analyses of anti-CD19 receptor and NKp30 receptor expressions for CAR-T cells of Comparative Example 1C are shown in Fig. 4E and Fig. 4F, respectively. The transduction efficiency of lentivirus for each group was determined based on the proportion of cells that expressed the anti-CD19 receptor, as listed in Table 4.

**Table 4: the transduction efficiencies of lentiviruses of Examples 1-1B, 1-4B and 1-5B (abbreviated as E1-1B, E1-4B and E1-5B), and Comparative Example 1B (abbreviated as CE1B)**

| Group | E1-1B | E1-4B | E1-5B | CE1B |
|---|---|---|---|---|
| Transduction efficiency | 10% | 50% to 60% | 25% to 40% | 50% to 85% |

When the transduction efficiency of preparing CAR-T cells was greater than 25%, it was beneficial to the subsequent functional tests of expanded CAR-T cells. Therefore, according to the results in Table 4, both the lentiviruses of Example 1-4B, having the IgG4 hinge domain, and of Example 1-5B, having the (EAAAK)₃ hinge domain, exhibited transduction efficiency that met the aforementioned criteria.

### Test Example 4: T cell subpopulation analysis for CAR-T cells of Examples 1-1C, 1-4C and 1-5C, and Comparative Example 1C

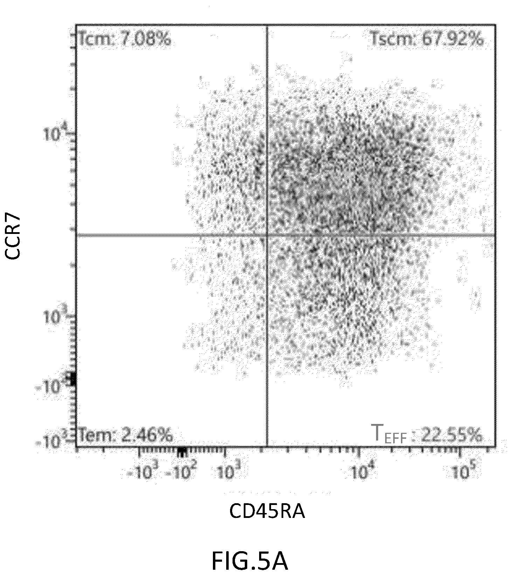

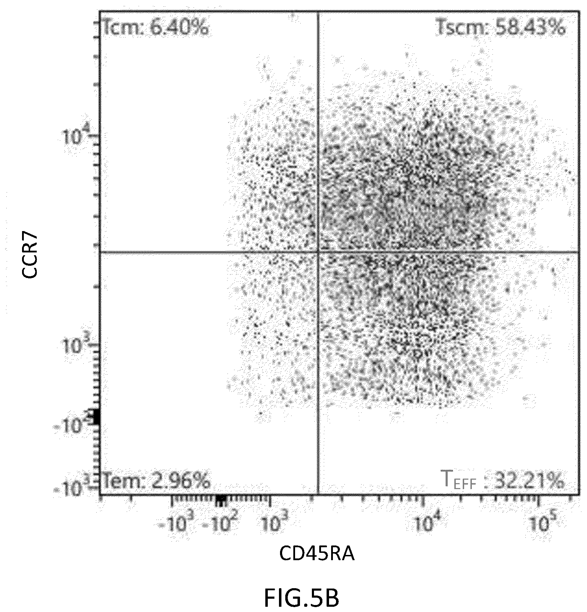

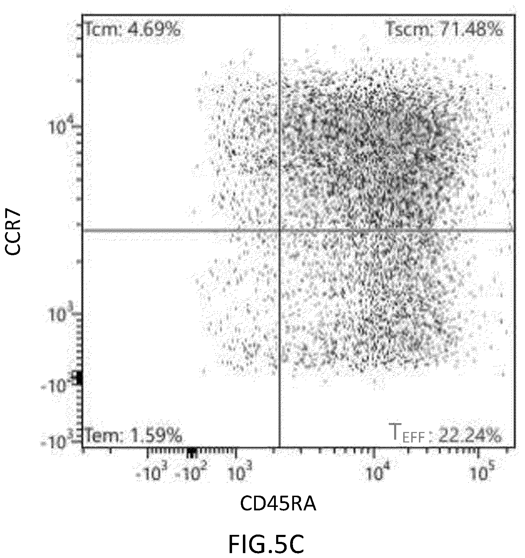

During the preparation of CAR-T cells with anti-CD19-NKp30 of Examples 1-1C, 1-4C and 1-5, the T cell subpopulation of CAR-T cells on day 7 were assessed by detecting cell-surface markers of T cells that were fluorescently labeled by antibodies and a flow cytometer with SA3800 software (purchased from Sony Biotechnology); wherein, the used antibodies were CD3-FITC (purchased from BioLegend, 300406), CD4-PE-Cy7 (purchased from BioLegend, 300512), CD8-AF700 (purchased from Beckman, B76279), CD95-APC (purchased from BioLegend, 305612), CD45RA-PE (purchased from BioLegend, 304108) and CCR7-BV421 (purchased from BioLegend, 353208). A fluorescence-minus-one (FMO) control group was also included for flow cytometric analysis, which meant that samples were stained with all foresaid fluorescently labeled antibodies except for CCR7-BV421 and CD45RA-PE. In this Test Example, un-transduced, activated T cells were used as a control group (an un-transduced group). The staining results obtained from the foresaid method were used to determine the distribution in percentage of each T cell subpopulation: stem-cell like memory T cell (Tscm), central memory T-cell (T_{CM}), effector memory T-cell (T_{EM}), or terminally differentiated effector memory T-cell [T_{EMRA}; also named as effector T cell (T_{EFF})] in accordance with Annals of Oncology Volume 32, Issue 11, Pages 1366-1380. The percentages of Tscm in CAR-T cells of Examples 1-1C, 1-4C and 1-5C, and Comparative Example 1C are shown in Table 5. The distribution of the subpopulations of T cells for Example 1-5C, Comparative Example 1C and the un-transduced group, analyzed by flow cytometry, are shown in Figs. 5A, 5B, and 5C, respectively; wherein, T cells simultaneously expressing CD45RA and CCR7 were considered Tscm, T cells expressing CD45RA but not expressing CCR7 were considered T_{EMRA} (also named as T_{EFF}), T cells not expressing CCR7 and CD45RA were considered T_{EM}, and T cells expressing CCR7 but not expressing CD45RA were considered T_{CM}.

**Table 5: the percentages of Tscm for CAR-T cells of Examples 1-1C, 1-4C and 1-5C (abbreviated as E1-1C, E1-4C and E1-5C) and Comparative Example 1C (abbreviated as CE1C), and for T cells of the un-transduced group (abbreviated as UTD)**

| Group | E1-1C | E1-4C | E1-5C | CE1C | UTD (control group) |
|---|---|---|---|---|---|
| Percentage Tscm | 40% | 40% to 70% | 40% to 70% | 40% to 70% | 71.48 % |

The desirable range for percentage Tscm of CAR-T cells was greater than 40%, because CAR-Ts with high proportions of Tscm were associated with reduced cytokine release syndrome and neurotoxicity caused by such cell therapy. According to the results in Table 5 and Figs. 5A to 5C, Example 1-1C (which had the (G₄S)₃ hinge domain), Example 1-4C (which had the IgG4 hinge domain), and Example 1-5C (which had the (EAAAK)₃ hinge domain) all displayed high proportions of Tscm, which were comparable to the traditional single-chain CAR-T cells of Comparative Example 1C. Therefore, it can be expected that CAR-T cells of Examples 1-1C, 1-4C and 1-5C were able to reduce cytokine release syndrome and neurotoxicity caused by such therapy.

### Test Example 5: Cytotoxicity of CAR-T cells of Examples 1-4C and 1-5C determined based on luciferase killing assay

To establish a luciferase-expressing Raji cell line, lentivirus vector PLL-CMV-rFluc-T2A-GFP-mPGK Lenti-Labeler (purchased from System Biosciences) was transduced into a Raji human lymphoma cell line (purchased from Elabscience, EP-CL-0189; hereinafter referred to as Raji cells). Then, single cell lines capable of stable expression was screened out to obtain GFP/Luc⁺ Raji cells that could simultaneously express green fluorescent protein (GFP) and a firefly luciferase enzyme. Because Raji cells constitutively express CD19, the GFP/Luc⁺ Raji cells were cultured in RPMI-1640 medium (purchased from Gibco) containing 10% FBS and adopted as the target cell for anti-CD19 CAR-T luciferase killing assay.

In order to determine the anti-tumor activities of CAR-T cells prepared according to the 7 days manufacturing method with anti-CD19-NKp30 CAR expression of Examples 1-4C and 1-5C, the foresaid GFP/Luc⁺ Raji cells, which expressed CD19 constitutively, were adopted as the target cells to conduct the luciferase killing assay. The effector cells used in this Test Example were CAR-T cells of Examples 1-4C and 1-5C, and Comparative Example 1C, and un-transduced T cells were used as an un-transduced group (a control group). Specifically, the effector cells and the target cells were co-cultured in 96-well microplates at 37°C in 5% CO₂ for 24 hours with an effector-to-target ratio (E:T ratio) of 3:1, and with the experiment performed in triplicates.

Luciferase Assay System (purchased from Promega, E1501) was used to quantify firefly luciferase activity, displayed by viable luciferase-expressing Raji cells, by providing luciferin for bioluminescent reaction. The relative numbers of surviving cancer cells, not lysed by CAR-T cells, can be evaluated based on luciferase detection. Consequently, the results of specific cell lysis ratios were calculated using the following equation: specific cell lysis ratio (%) = 100% × [(number of lysed target cells due to treatment) - (number of spontaneously lysed target cells)] / [(maximum number of lysed target cells) - (number of spontaneously lysed target cells)];
wherein, the "number of lysed target cells due to treatment" = the number of lysed target cells obtained from the group of co-culturing the target cells with CAR-T cells (*i.e.,* effector cells);
the "number of spontaneously lysed target cells" = the number of lysed target cells obtained from the group in the absence of effector cells, *i.e.,* CAR-T cells, which represented the number of lysed cells simply by culturing the GFP/Luc⁺ Raji cells in RPMI-1640 medium containing 10% FBS at 37°C in 5% CO₂ for 24 hours;
the "maximum number of lysed target cells" = the number of lysed target cells obtained after 100 µL of 1% Triton X-100 was added into the group in the absence of effector cells.

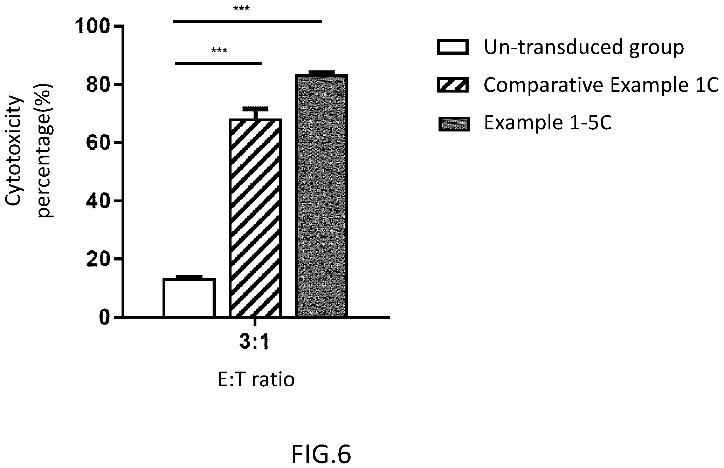

The experimental results are shown in Table 6; and, the percentage of cytotoxicity indicated by the foresaid specific cell lysis ratio of CAR-T cells of Example 1-5C, CAR-T cells of Comparative Example 1C and T cells of the un-transduced group are shown in Fig. 6.

**Table 6: the results of luciferase killing assay (specific cell lysis ratio) of CAR-T cells of Examples 1-4C and 1-5C (abbreviated as E1-4C and E1-5C), and Comparative Example 1C (abbreviated as CE1C), and T cells of the un-transduced group (abbreviated as UTD)**

| Group | E1-4C | E1-5C | CE1C | UTD (control group) |
|---|---|---|---|---|
| Specific cell lysis ratio (at E:T=3:1) | 51% | 83.6% | 60% to 70% | 27% |

According to the results in Table 6 and Fig. 6, both the cytotoxicity of CAR-T cells of Examples 1-4C and 1-5C were better than the un-transduced group, and the cytotoxicity of CAR-T cells of Example 1-5C was even better than that of Comparative Example 1C. Besides, when the cytotoxicity of CAR-T cells during text production exceeded 50%, CAR-T produced using patient's cells should also exhibit sufficient cytotoxicity. According to the results in Table 6, both CAR-T cells of Examples 1-4C and 1-5C demonstrated cytotoxicity that met the >50% criteria, whilst the cytotoxicity of CAR-T cells of Example 1-5C (which had the (EAAAK)₃ hinge domain) was even better than that of traditional single-chain CAR-T cells of Comparative Example 1C.

### Test Example 6: Real-time cytotoxicity assay (RTCA) for the analyses of Example 1-5C and Comparative Example 1C against CD19-expressing adherent cancer cells

Human CD19 cDNA fragment was cloned from Raji cells (purchased from Elabscience, CL-0189). The cDNA fragment encoding truncated CD19 (shown as SEQ ID NO: 90) was amplified by PCR; wherein, the foresaid truncated CD19 cDNA fragment (amino acid 1 to 342) indicated the cDNA fragment having extracellular and transmembrane domains but lacking intracellular domain and cytoplasmic signaling domains. The foresaid truncated CD19 cDNA fragment was ligated into a pLAS5w.Ppuro vector, containing an anti-puromycin gene (purchased from Academia Sinica RNAi Core facility, Product No.: C6-8-39), using restriction enzyme sites of HpaI and NheI, which following completion was then transfected into SKOV3 cells (human ovarian cancer cell line) by applying Polyjet transfection reagent (purchased from SignaGen Laboratories, SL100688). The truncated CD19 transfected SKOV3 cells were cultured in McCoy 5A Medium (purchased from Gibco, 16600082) containing 2 µg/mL of puromycin and 10% FBS to select a SKOV3 cell line that was puromycin-resistant and expressed CD19 at the same time. At last, the expression of CD19 was re-checked by flow cytometric analysis. The SKOV3 cell line expressing CD19 was used as target cells for the real-time cytotoxicity assay conducted using the xCELLigence system (hereinafter referred to as xCELLigence RTCA system).

The xCELLigence RTCA system (purchased from Agilent Technologies, RTCA SP) was placed in a cell incubator at 37°C in 5% CO₂, and connected to the interfaces of an external analysis unit and a control unit by cables; meanwhile, the RTCA Software Pro software possessed functions of real-time controlling and monitoring of instruments, including real-time data display and analysis. Specifically, the xCELLigence RTCA system was used to continuously monitor changes in the cytotoxicity of treatment cells on tumor cells for 90 hours. McCoy 5A Medium containing 10% FBS was added into each well of 96-well microplate (E-plate, Agilent Technologies, 300600910) to culture cancer cells. After shortly measuring the background electrical impedance of the medium, the foresaid SKOV3 cells expressing truncated CD19 were used as target cells and seeded at a cell density of 2×10⁴ cells/mL into the foresaid microplate with the background impedance corrected, and left overnight to ensure that the target cells (T) attached. To allow sufficient cancer cell growth, the background impedance measurement was performed for a total of 29 hours, at which time, effector cells (E) were then added into the microplate for co-culture at an effector cell to target cell ratio (E:T ratio) of 0.1:1 with the final volume of each well being 200 µL. The effector cells used in this Test Example were CAR-T cells of Example 1-5C and Comparative Example 1C. In addition, un-transduced T cells were used as an un-transduced group (a control group), and the target cells treated with 100 µL of 1% Triton X-100 were used as a whole lysis group. The cell index (CI) was measured every 15 minutes for at least 72 hours. The raw RTCA impedance background data were normalized with the measured cell index value before addition of the effector cells. The foresaid cell index was a dimensionless parameter and obtained by measuring the relative change of electrical impedance, which represented the status of cells. For example, when cells did not exist or were unattached on the electrodes, the cell index was zero; and under the same physiological conditions, the more the number of cells attached on the electrodes, the higher the value of cell index. Nevertheless, status changes of cells, such as changes in cell morphology or cell adhesion, could change the cell index.

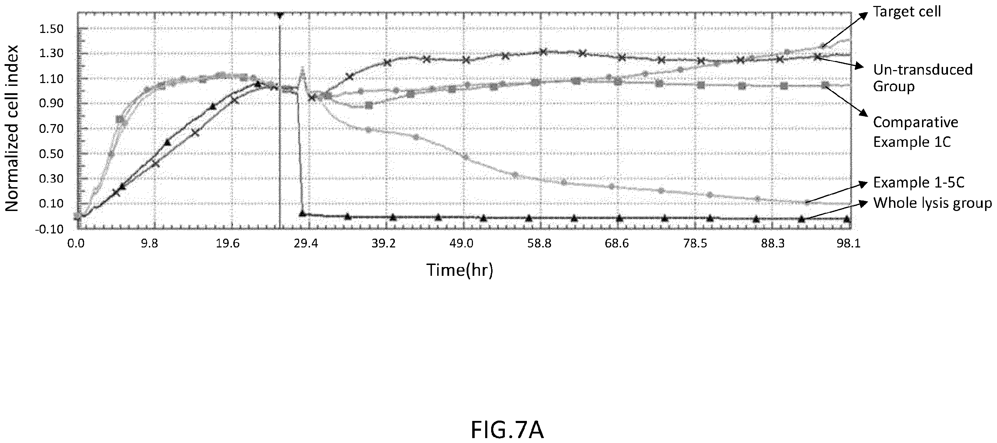

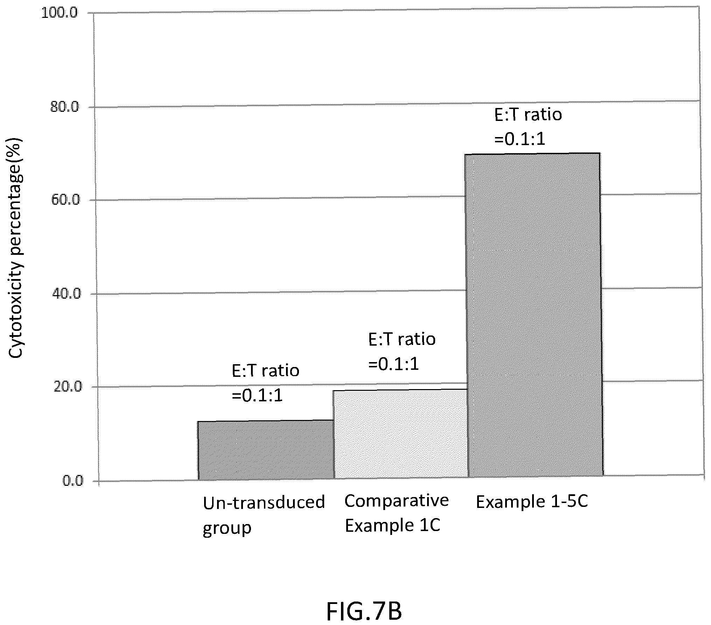

The experimental results are shown in Fig. 7A and Fig. 7B, which showed that the cytotoxicity of CAR-T cells of Example 1-5C (which had the (EAAAK)₃ hinge domain) was significantly better than that of CAR-T cells of Comparative Example 1C and the T cells of the un-transduced group, and was over three times more than the cytotoxicity of CAR-T cells of Comparative Example 1C, thereby showing excellent cytotoxicity.

In summary, the lentivirus of Example 1-5C (which had the (EAAAK)₃ hinge domain) had reasonable transduction efficiency, and CAR-T cells of Example 1-5C expanded well, exhibited excellent cytotoxicity and higher proportions of Tscm, which could potentially lead to the reduction in cytokine release syndrome and neurotoxicity caused by such therapy. Therefore, the (EAAAK)₃ hinge domain was the most suitable hinge domain to match with the anti-CD19 receptor.

### II. Production and characterization of CAR-T cells expressing anti-mesothelin-NKp30

### Example 2: Anti-mesothelin-NKp30 nucleic acid molecule design

The preparation method of Example 2 was similar to the preparation method of Example 1-1, except that the single-chain variable fragment used in Example 2 was not FMC63 scFv for anti-CD19 used in the preparation method of Example 1-1, but a nucleic acid sequence encoding SS1 scFv capable of targeting mesothelin (shown as SEQ ID NO: 91), and the adopted hinge domain was (G₄S)₃. A nucleic acid sequence of the nucleic acid molecule of Example 2 is shown as SEQ ID NO: 92.

The preparation method of Comparative Example 2, which did not contain NKp30, was similar to the preparation method of Comparative Example 1, except that the single-chain variable fragment used in Comparative Example 2 was not FMC63 scFv for anti-CD19 used in the preparation method of Comparative Example 1, but a nucleic acid sequence encoding SS1 scFv capable of targeting mesothelin. A nucleic acid sequence of the nucleic acid molecule of Comparative Example 2 is shown as SEQ ID NO: 93.

### Example 2A: Construction of anti-mesothelin-NKp30 plasmids

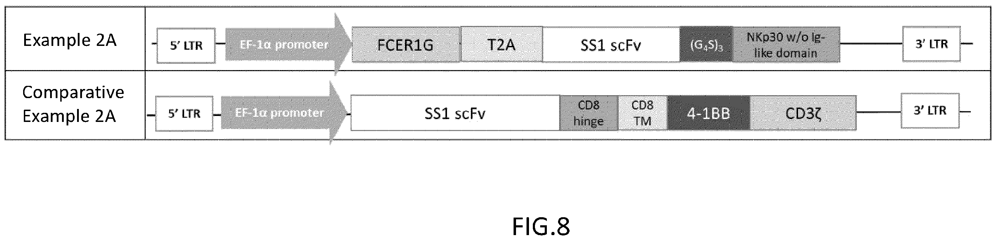

The preparation method of Example 2A was similar to the preparation method of plasmid of Example 1-1A, except that the anti-mesothelin-NKp30 nucleic acid molecule of Example 2 was ligated into the foresaid pLAS5w vector by restriction enzymes of BstBI and EcoRI. While, the nucleic acid molecule of Comparative Example 2 was ligated into pLAS5w.Ppuro vector (purchased from Academia Sinica RNAi Core facility, Product No.: C6-8-39) by restriction enzymes of HpaI and EcoRI to obtain the plasmid of Comparative Example 2A (conducted by GenScript). The prepared anti-mesothelin-NKp30 plasmid of Example 2A and the plasmid of Comparative Example 2A are shown in Fig. 8.

### Example 2B: Production of anti-mesothelin-NKp30 lentiviruses

The preparation method of Example 2B was similar to the preparation method of Example 1-1B, except that the plasmid of Example 2A was adopted as the transfer plasmid of Example 2B. While, the plasmid of Comparative Example 2A was adopted as the transfer plasmid for the lentivirus of Comparative Example 2B.

### Test Example 7: Titer determination of anti-mesothelin-NKp30 lentiviruses of Example 2B

The method of Test Example 7 was similar to that of Test Example 1, except that Biotinylated Human Mesothelin, His, Avitag (purchased from ACROBiosystems, MSN-H82E9) was used to target anti-mesothelin receptor and 500-fold diluted Streptavidin conjugated PE (purchased from Invitrogen, 12-4317-87) was used to detect the expression of anti-mesothelin receptor. The results of Test Example 7 are shown in Table 7.

**Table 7: the functional viral titer of lentiviruses of Example 2B (abbreviated as E2B) and Comparative Example 2B (abbreviated as CE2B)**

| Group | E2B | CE2B |
|---|---|---|
| Functional viral titer (TU/mL) | 3.9×10⁷ | 3×10⁷ to 7×10⁷ |

When the functional viral titer was more than or equal to 1×10⁶ TU/mL, T cells could be successfully transduced and become CAR-T cells with a lower amount of lentivirus, whereas, a lower volume of lentivirus used during transduction also tended to correlate with better growth of T cells for CAR-T production. Therefore, according to the results in Table 7, Example 2B (which had the (G₄S)₃ hinge domain) met the foresaid criteria, which further benefited preparation of CAR-T cells.

### Example 2C: Manufacturing CAR-T cells that express anti-mesothelin-NKp30

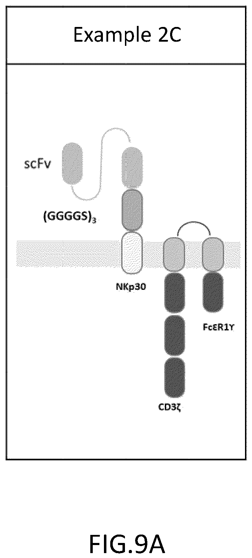

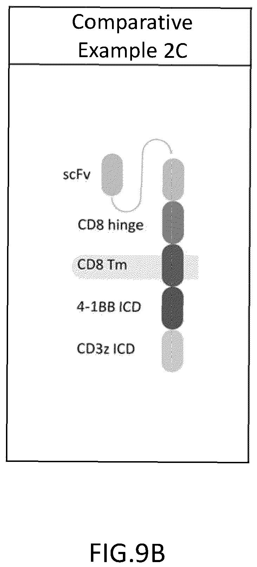

The preparation method of CAR-T cells with anti-mesothelin-NKp30 of Example 2C was similar to the preparation method of Example 1-1C, except that the T cells were transduced with the lentivirus of Example 2B and the MOI of lentivirus transduction was 3. While, CAR-T cells of Comparative Example 2C were prepared by transducing with the lentivirus of Comparative Example 2B and the MOI of lentivirus transduction was 3. The structural schematic illustrations of CAR-T cells with anti-mesothelin-NKp30 of Example 2C and CAR-T cells of Comparative Example 2C are shown in Fig. 9A and Fig. 9B, respectively.

### Test Example 8: Cell expansion rate of CAR-T cells with anti-mesothelin-NKp30

The method of Test Example 8 was similar to that of Test Example 2, except that the cell expansion rate of CAR-T cells with anti-mesothelin-NKp30 of Example 2C was detected, of which results are shown in Table 8.

**Table 8: fold of cell expansion of CAR-T cells of Example 2C (abbreviated as E2C) and Comparative Example 2C (abbreviated as CE2C)**

| Group | E2C | CE2C |
|---|---|---|
| Fold of cell expansion (day 7 relative to day 0) | 29.4-fold | 18.9-fold |

When the cell expansion rate was greater than 5-fold, it was beneficial to the subsequent functional tests of T cells. Therefore, according to the results in Table 8, the cell expansion rate for CAR-T cells of Example 2C met such criteria, which appeared to be greater than that of CAR-T cells of Comparative Example 2C.

### Test Example 9: Resulting CAR-positive expression of CAR-T cells of Example 2C and Comparative Example 2C, indicating transduction efficiencies of lentiviruses of Example 2B and Comparative Example 2B

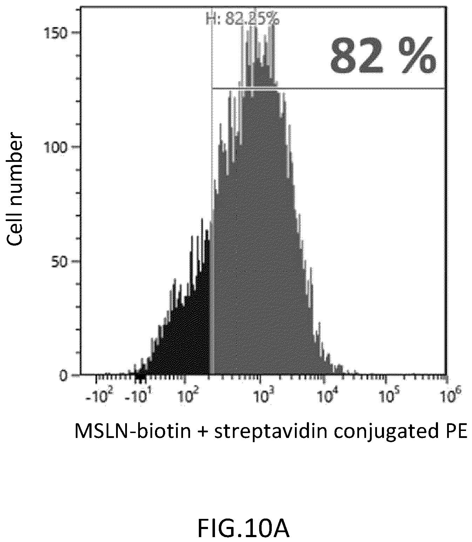

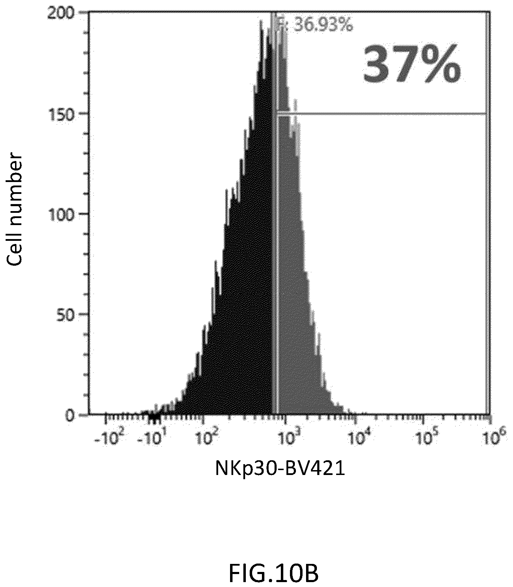

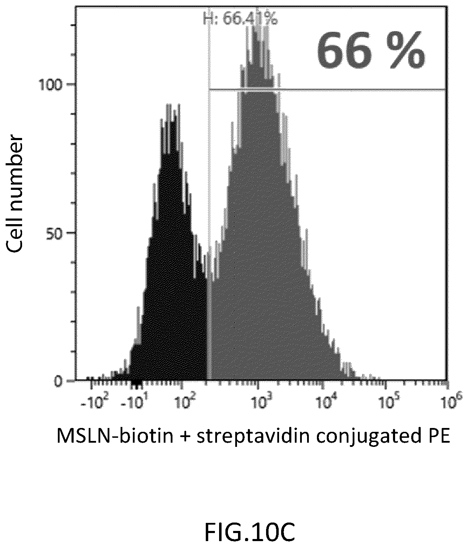

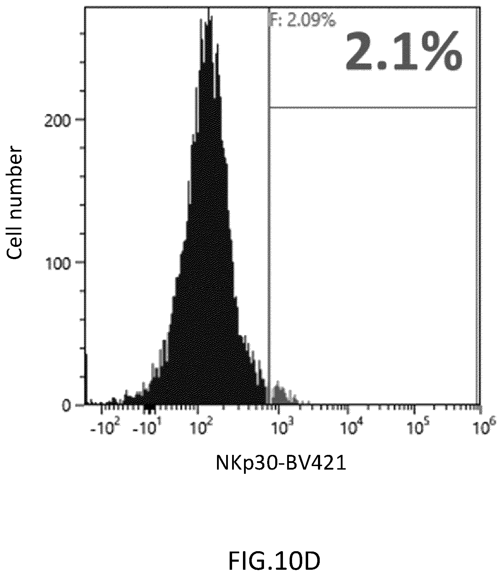

The method of Test Example 9 was similar to that of Test Example 3, except that Biotinylated Human Mesothelin, His, Avitag (purchased from ACROBiosystems, MSN-H82E9) was used to target anti-mesothelin receptor and 500-fold diluted Streptavidin conjugated PE (purchased from Invitrogen, 12-4317-87) was used to detect the expressions of anti-mesothelin receptor and NKp30 receptor expressions of CAR-T cells of Example 2C and Comparative Example 2C. Flow cytometric analysis results of Example 2C and Comparative Example 2C are shown in Fig. 10A, Fig. 10B, Fig. 10C and Fig. 10D, respectively. The transduction efficiencies of lentiviruses for Example 2B or Comparative Example 2B were determined based on the proportion of cells that expressed the anti-mesothelin receptor, as listed in Table 9.

**Table 9: the transduction efficiencies of lentiviruses of Example 2B (abbreviated as E2B) and Comparative Example 2B (abbreviated as CE2B)**

| Group | E2B | CE2B |
|---|---|---|
| Transduction efficiency | 50% to 85% | 60% to 79% |

When the transduction efficiency of preparing CAR-T cells was greater than 25%, it was beneficial to the subsequent functional tests of expanded CAR-T cells. Therefore, according to the results in Table 9 and Fig. 10A, the lentivirus of Example 2B exhibited transduction efficiency that met the aforementioned criteria.

### Test Example 10: T cell subpopulation analysis for CAR-T cells of Example 2C

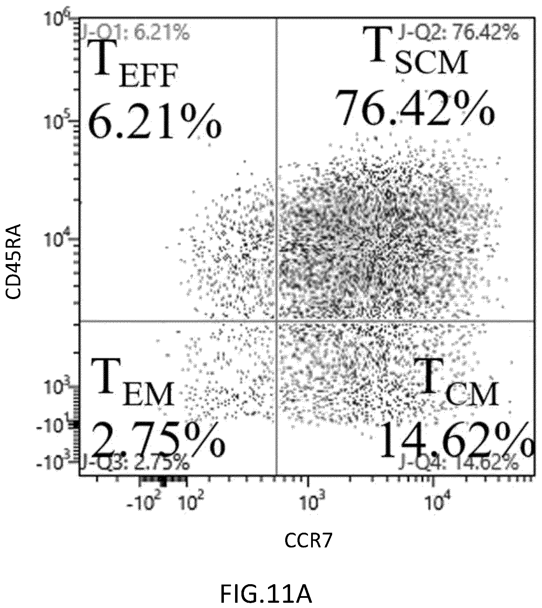

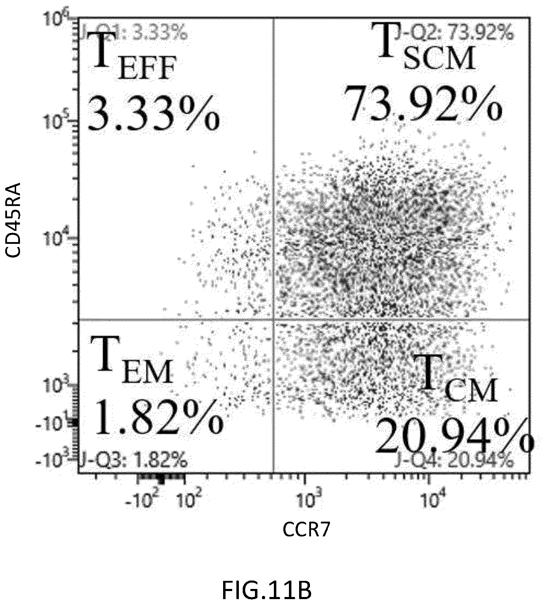

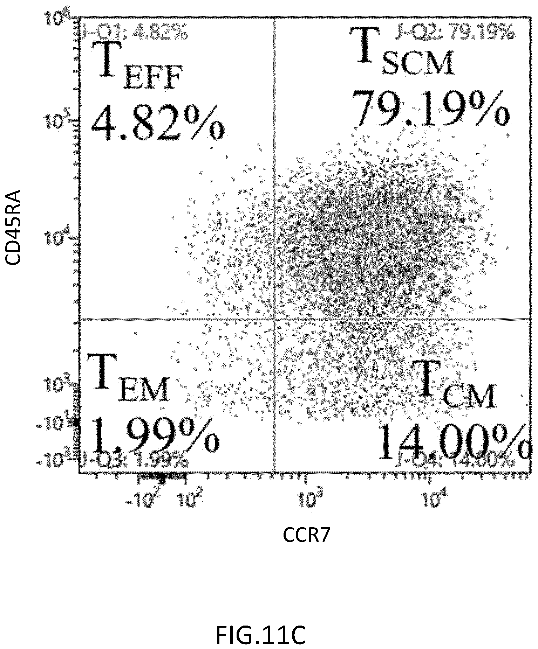

The method of Test Example 10 was similar to that of Test Example 4, except that the analyses were performed on day 7 of the preparation of CAR-T cells with anti-mesothelin-NKp30 of Example 2C. The percentages of Tscm in CAR-T cells of Example 2C and Comparative Example 2C, and in T cells of the un-transduced group are shown in Table 10; and the distribution of the subpopulations of T cells of Example 2C, Comparative Example 2C and the un-transduced group analyzed by flow cytometry are shown in Fig. 11A, Fig. 11B and Fig. 11C, respectively; wherein, T cells simultaneously expressing CD45RA and CCR7 were considered Tscm.

**Table 10: the percentages of Tscm of Example 2C (abbreviated as E2C), and Comparative Example 2C (abbreviated as CE2C), and the percentage of Tscm in activated T cells of the un-transduced group (abbreviated as UTD)**

| Group | E2C | CE2C | UTD |
|---|---|---|---|
| Percentage Tscm | 76% | 74% | 79% |

When the percentage of Tscm in CAR-T cells was greater than 40%, cytokine release syndrome and neurotoxicity caused by CAR-T cell therapy could potentially be reduced. According to the results in Table 10 and Fig. 11A, Fig. 11B and Fig. 11C, Example 2C (which had the (G₄S)₃ hinge domain) displayed high proportions of Tscm, which were greater than that of traditional single-chain CAR-T cells of Comparative Example 2C, while similar to that of activated T cells of the un-transduced group. Therefore, it could be expected that CAR-T cells of Example 2C were potentially capable of reducing cytokine release syndrome and neurotoxicity caused by such therapy.

### Test Example 11: Real-time cytotoxicity assay for the analyses of Example 2C and Comparative Example 2C against Hela cancer cells

Hela cervical cancer cell line (hereinafter referred to as Hela cells) was a mesothelin-positive cell line, and Hela cells (purchased from Elabscience, CL-0101) were cultured in DMEM containing 10% FBS at 37°C in 5% CO2 to be used as target cells in subsequent real-time cytotoxicity assay performed on the xCELLigence system.

The method of Test Example 11 was similar to that of Test Example 6, except that the effector cells were CAR-T cells of Example 2C or Comparative Example 2C, and the target cells were the foresaid Hela cells. The target cells with a cell density of 1.2×10⁴ cells/mL were seeded in a 96-well microplate with the background impedance corrected, followed by 18 hours of overnight culture to ensure that the target cells attach. Then, the effector cells were added into the microplate at E:T ratios of 3:1, 1:1 and 0.3:1. Test Example 11 did not adopt a whole lysis group as a control group.

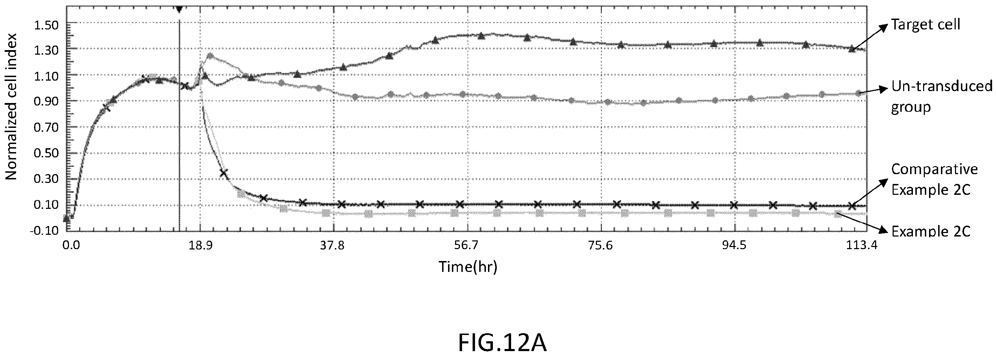

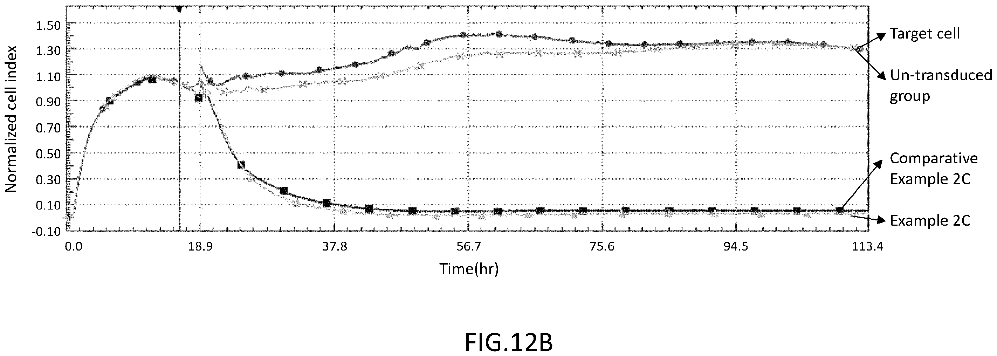

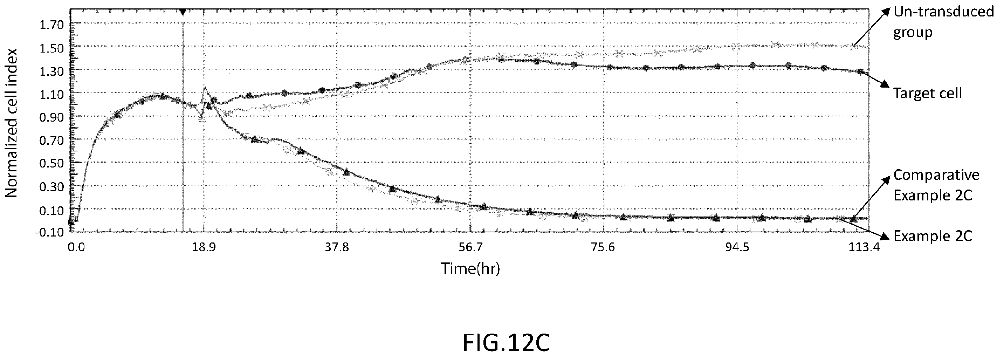

The experimental results of different E:T ratios are shown in Fig. 12A to Fig. 12C, which demonstrate that at 58 hours into the experiment, *i.e.,* after a co-culture period of 40 hours, CAR-T cells of Example 2C could kill 100% of Hela cells at all three E:T ratios. Meanwhile, the cytotoxicity of CAR-T cells of Example 2C was comparable to that of single-chain CAR-T cells of Comparative Example 2C.

### Test Example 12: Re-challenge test of real-time cytotoxicity assay by using xCELLigence system

The objective of Test Example 12 was to test whether the effector cells could exhibit persistent cytotoxicity if they were continuously re-stimulated by the target cells. DMEM containing 10% FBS was added into each well of 96-well microplate (E-plate, Agilent Technologies, 300600910). After shortly measuring the background electrical impedance of the medium, Hela cells (purchased from Elabscience, CL-0101) were used as the target cells, and were cultured in the 96-well microplate with DMEM containing 10% FBS. The target cells were seeded at a cell density of 1.2×10⁴ cells/mL into the 96-well microplate with its background impedance corrected, and left to culture overnight to enable the target cells attachment. To allow sufficient cancer cell growth, the background impedance measurement was performed for 18 hours, at which time the effector cells were then added into the microplate at an E:T ratio of 1:1. Hela cells were re-supplied daily (2.4×10⁴ Hela cells on both the second and third day, respectively) for the tumor re-challenge assay. Test Example 12 adopted CAR-T cells of Example 2C and Comparative Example 2C as the effector cells, and un-transduced T cells were used as the un-transduced group (a control group). The cell index was measured every 15 minutes for at least 72 hours, and the raw RTCA impedance data were normalized with the measured cell index value prior to adding the effector cells.

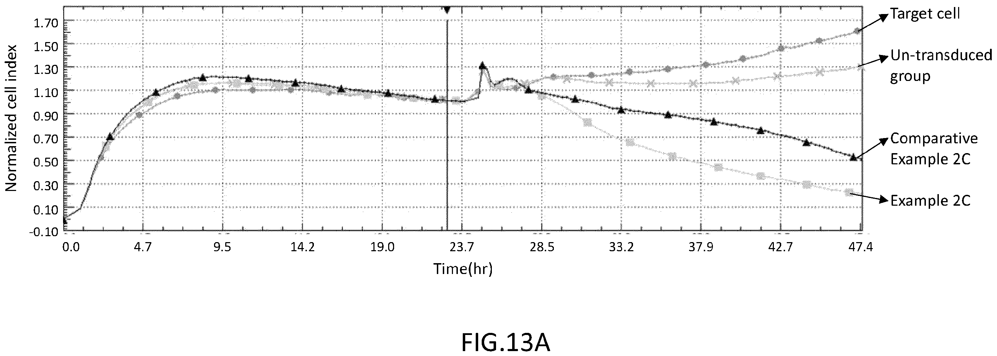

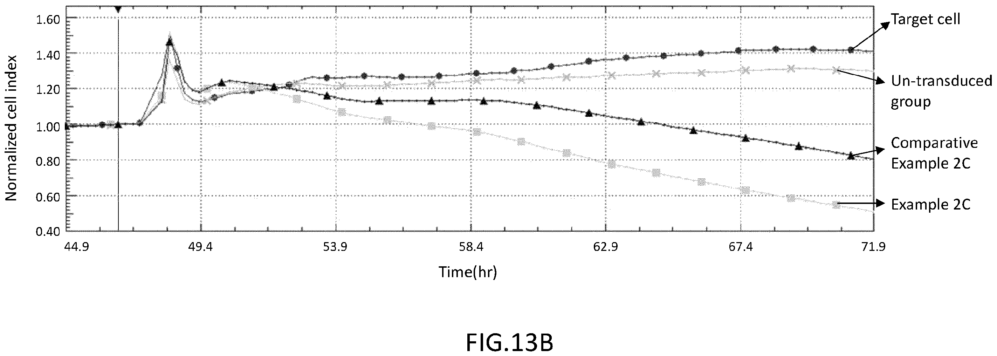

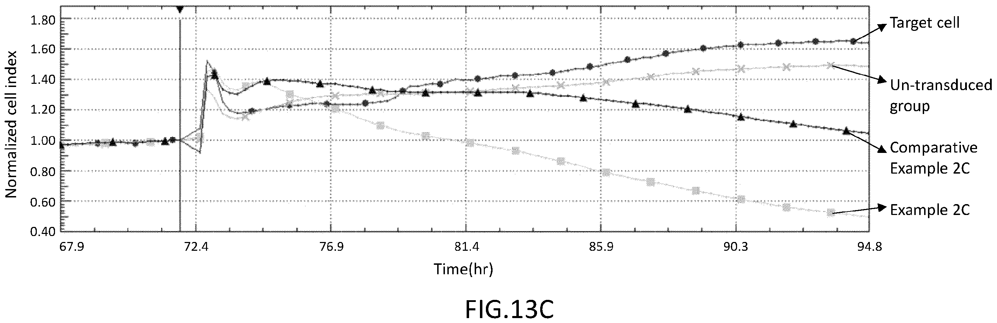

The real-time cytotoxicity results of CAR-Ts against Hela cancer cells for the first day and following cancer cell re-challenge, on the second and the third days, are respectively shown in Fig. 13A, Fig. 13B and Fig. 13C. Cytotoxicity of Example 2C was greater than Comparative Example 2C prior to and following both days of cancer cell re-challenge. Therefore, Example 2C not only exhibited excellent cytotoxicity against cancer cells, but such cytotoxicity effects were also found to be persistent. Accordingly, Test Example 12 confirmed that both the degree of cytotoxicity as well as its persistency of CAR-T cells of Example 2C were greater than those of CAR-T cells of Comparative Example 2C.

In summary, the lentivirus of Example 2B (which had the (G₄S)₃ hinge domain) had decent transduction efficiency, and CAR-T cells of Example 2C expanded well, exhibited strong and persistent cytotoxicity, and possessed higher proportions of Tscm, which can potentially lead to the reduction in cytokine release syndrome and neurotoxicity caused by such therapy. Therefore, the (G₄S)₃ hinge domain was a suitable hinge domain to match with the anti-mesothelin receptor.

### III. Production and characterization of CAR-T cells expressing anti-BCMA-NKp30

### Example 3: Anti-BCMA-NKp30 nucleic acid molecule design

The preparation method of Example 3 was similar to the preparation method of Example 1-1, except that the single-chain variable fragment used in Example 3 was not FMC63 scFv for anti-CD19 used in the preparation method of Example 1-1, but a nucleic acid sequence encoding J6M0 scFv capable of targeting BCMA (shown as SEQ ID NO: 94), and the adopted hinge domain was the hinge domain of CD28. A nucleic acid sequence of the nucleic acid molecule of Examples 3 is shown as SEQ ID NO: 95.

The preparation method of Comparative Example 3, which did not contain NKp30, was similar to the preparation method of Comparative Example 1, except that the single-chain variable fragment used in Comparative Example 3 was not FMC63 scFv for anti-CD19 used in the preparation method of Comparative Example 1, but a nucleic acid sequence encoding J6M0 scFv capable of targeting BCMA. A nucleic acid sequence of the nucleic acid molecule of Comparative Example 3 is shown as SEQ ID NO: 96.

### Example 3A: Construction of anti-BCMA-NKp30 plasmids

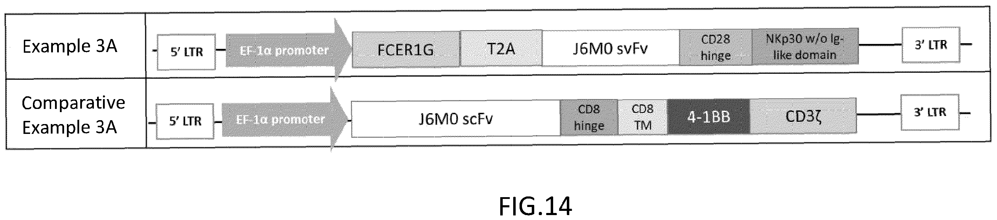

The preparation method of Example 3A was similar to the preparation method of plasmid of Example 1-1A, except that the anti-BCMA-NKp30 nucleic acid molecule of Example 3 was ligated into the foresaid pLAS5w vector by restriction enzymes of NheI and EcoRI. While, the nucleic acid molecule of Comparative Example 3 was ligated into pLAS5w.Ppuro vector (purchased from Academia Sinica RNAi Core facility, Product No.: C6-8-39) by restriction enzymes of HpaI and EcoRI to obtain the plasmid of Comparative Example 3A (conducted by GenScript). The prepared anti-BCMA-NKp30 plasmid of Example 3A and the plasmid of Comparative Example 3A are shown in Fig. 14.

### Example 3B: Production of anti-BCMA-NKp30 lentiviruses

The preparation method of Example 3B was similar to the preparation method of Example 1-2B, except that the plasmid of Example 3A was adopted as the transfer plasmid of Example 3B. While, the plasmid of Comparative Example 3A was adopted as transfer plasmid for the lentivirus of Comparative Example 3B.

### Test Example 13: Titer determination of anti-BCMA-NKp30 lentiviruses of Example 3B

The method of Test Example 13 was similar to that of Test Example 1, except that Biotinylated BCMA Protein, His, Avitag (His & AVI Tag, purchased from Sino Biological, Cat: 10620-H40H-B) was used to target anti-BCMA receptor and 500-fold diluted Streptavidin conjugated PE (purchased from Invitrogen, 12-4317-87) was used to detect the expression of anti-BCMA receptor. The results of Test Example 13 are shown in Table 11.

**Table 11: the functional viral titer of lentiviruses of Example 3B (abbreviated as E3B) and Comparative Example 3B (abbreviated as CE3B)**

| Group | E3B | CE3B |
|---|---|---|
| Functional viral titer (TU/mL) | 1.44×10⁷ | 1.5×10⁷ |

When the functional viral titer was more than or equal to 1×10⁶ TU/mL, T cells could be successfully transduced and become CAR-T cells with a lower amount of lentivirus, whereas, a lower volume of lentivirus used during transduction also tended to correlate with better growth of T cells for CAR-T production. Therefore, according to the results in Table 11, Example 3B (which had the CD28 hinge domain) met the foresaid criteria, which further benefited preparation of CAR-T cells. Also of note, the functional viral titer of Example 3B was found to be similar to that of Comparative Example 3B.

### Example 3C: Manufacturing CAR-T cells that express anti-BCMA-NKp30

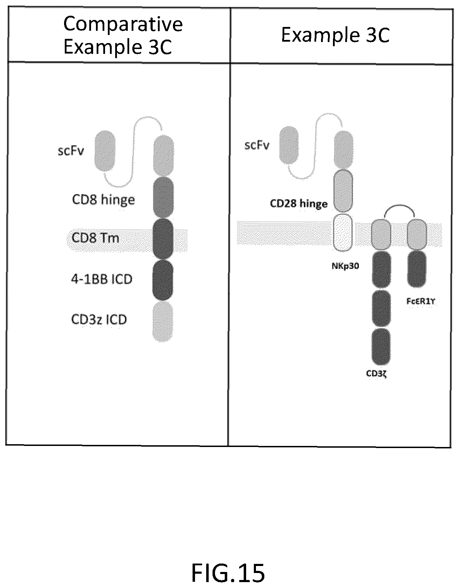

The preparation method of CAR-T cells with anti-BCMA-NKp30 of Example 3C was similar to the preparation method of Example 1-2C, except that the T cells were transduced with the lentivirus of Example 3B and the MOI of lentivirus transduction was 3. While, CAR-T cells of Comparative Example 3C were prepared by transducing with the lentivirus of Comparative Example 3B and the MOI of lentivirus transduction was 3. The structural schematic illustrations of CAR-T cells with anti-BCMA-NKp30 of Example 3C and CAR-T cells of Comparative Example 3C are shown in Fig. 15.

### Test Example 14: Cell expansion rate of CAR-T cells with anti-BCMA-NKp30

The method of Test Example 14 was similar to that of Test Example 2, except that the cell expansion rate of CAR-T cells with anti-BCMA-NKp30 of Example 3C was detected, of which results are shown in Table 12.

**Table 12: fold of cell expansion of CAR-T cells of Example 3C (abbreviated as E3C) and Comparative Example 3C (abbreviated as CE3C)**

| Group | E3C | CE3C |
|---|---|---|
| Fold of cell expansion (day 7 relative to day 0) | 20.2-fold | 12.5-fold |

When the cell expansion rate was greater than 5-fold, it was beneficial to the subsequent functional tests of T cells. Therefore, according to the results in Table 12, the cell expansion rate for CAR-T cells of Example 3C met such criteria, which was significantly greater than that of CAR-T cells of Comparative Example 3C.

### Test Example 15: Resulting CAR-positive expression of CAR-T cells of Example 3C and Comparative Example 3C, indicating transduction efficiencies of lentiviruses of Example 3B and Comparative Example 3B

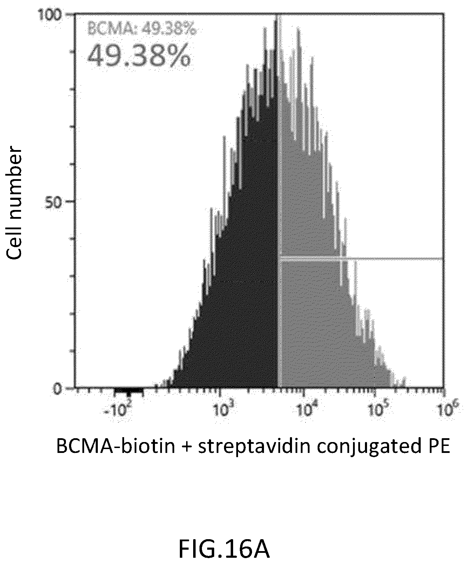

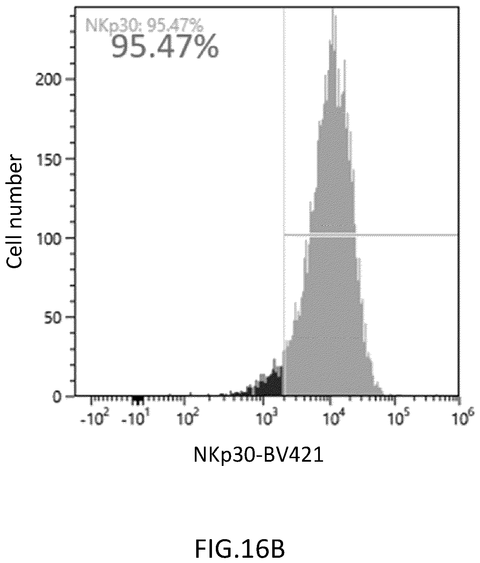

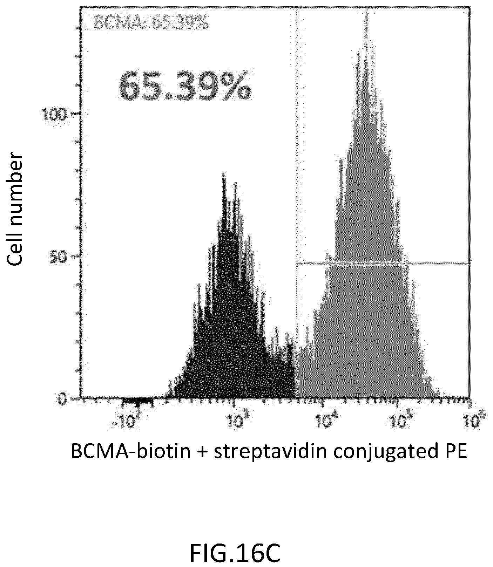

The method of Test Example 15 was similar to that of Test Example 3, except that Biotinylated BCMA Protein, His, Avitag (His & AVI Tag, purchased from Sino Biological, Cat: 10620-H40H-B) was used to target anti-BCMA receptor and 500-fold diluted Streptavidin conjugated PE (purchased from Invitrogen, 12-4317-87) was used to detect the expression of anti-BCMA receptor. The results of Example 3C and Comparative Example 3C analyzed by flow cytometry are shown in Fig. 16A, Fig. 16B and Fig. 16C, respectively. The transduction efficiencies of lentiviruses for Example 3B or Comparative Example 3B were determined based on the proportion of cells that expressed the anti-BCMA receptor, as listed in Table 13.

**Table 13: the transduction efficiencies of lentiviruses of Example 3B (abbreviated as E3B) and Comparative Example 3B (abbreviated as CE3B)**

| Group | E3B | CE3B |
|---|---|---|
| Transduction efficiency | 25% to 80% | 65% to 80% |

When the transduction efficiency of preparing CAR-T cells was greater than 25%, it was beneficial to the subsequent functional tests of expanded CAR-T cells. Therefore, according to the results in Table 13 and Fig. 16A, the lentivirus of Example 3B exhibited transduction efficiency that met the aforementioned criteria.

### Test Example 16: T cell subpopulation analysis for CAR-T cells of Example 3C

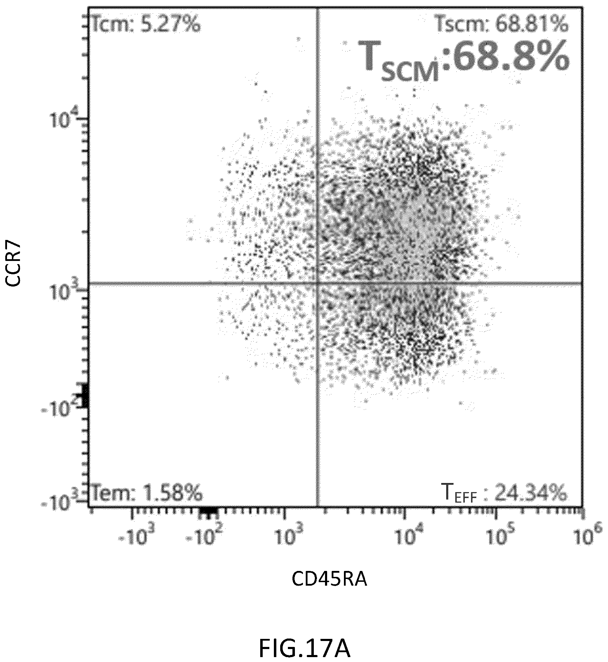

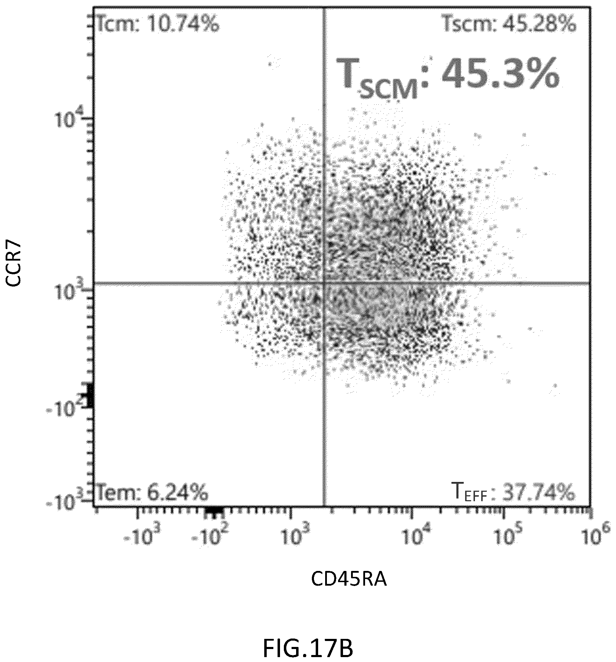

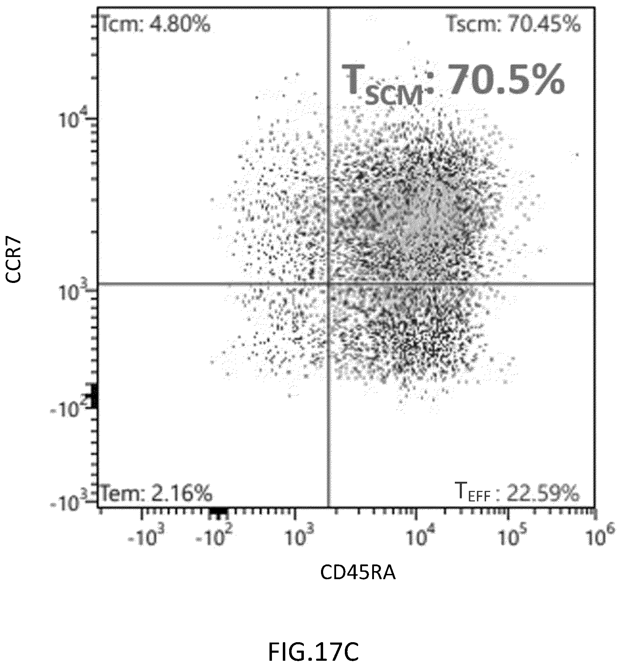

The method of Test Example 16 was similar to that of Test Example 4, except that the analyses were performed on day 7 of the preparation of CAR-T cells with anti-BCMA-NKp30 of Example 3C. The percentages of Tscm in CAR-T cells of Example 3C and Comparative Example 3C, and in T cells of the un-transduced group are shown in Table 14; and the distribution of the subpopulations of T cells of Example 3C, Comparative Example 3C and the un-transduced group analyzed by flow cytometry are shown in Fig. 17A, Fig. 17B and Fig. 17C, respectively; wherein, T cells simultaneously expressing CD45RA and CCR7 were considered Tscm.

**Table 14: the percentages of Tscm of Example 3C (abbreviated as E3C), and Comparative Example3C (abbreviated as CE3C), and the percentage of Tscm in T cells of the un-transduced group (abbreviated as UTD)**

| Group | E3C | CE3C | UTD |
|---|---|---|---|
| Percentage Tscm | 68.8% | 45.3% | 70.5% |

When the percentage of Tscm in CAR-T cells was greater than 40%, cytokine release syndrome and neurotoxicity caused by CAR-T cell therapy could potentially be reduced. According to the results in Table 14 and Fig. 17A, Fig. 17B and Fig. 17C, Example 3C (which had the hinge domain of CD28) displayed high proportions of Tscm, which were greater than that of traditional single-chain CAR-T cells of Comparative Example 3C, while similar to that of the activated T cells of the un-transduced group. Therefore, it could be expected that CAR-T cells of Example 3C were potentially capable of reducing cytokine release syndrome and neurotoxicity caused by such therapy.

### Test Example 17: Cytotoxicity assay analyzed by flow cytometry

In order to test the anti-tumor activity of CAR-T cells with anti-BCMA-NKp30, in Test Example 17, H929 myeloma cell line (purchased from BCRC, 60386; hereinafter referred to as H929 cell) was labeled with CellTrace^{™} Violet and cultured in RPMI-1640 medium (purchased from Gibco) to be used in cytotoxicity assay analyzed by flow cytometry. In Test Example 17, the effector cells were CAR-T cells of Example 3C and Comparative Example 3C, and un-transduced T cells were used as an un-transduced group (a control group). Specifically, the target cells with a cell density of 1×10⁴ cells/mL were seeded in 96-well microplates, followed by the addition of the effector cells into the microplates at E:T ratios of 0.1:1, 0.3:1 and 1:1. The co-culture was conducted at 37°C in 5% CO₂ for 24 hours, and with the experiment performed in triplicates. Following co-culture, the 96-well microplates were centrifuged at 300×g for 5 minutes to pellet the remaining cells and then the supernatant was removed. Cell pellets in each well were washed with 200 µL of PBS containing 2% fetal calf serum (FCS) and then centrifuged again; followed by removal of the supernatant from the washed cell pellet. 300 µL of flow buffer (PBS containing 2% FCS) was applied to resuspend and collect the cells. Fifteen microliters of counting microbeads (CountBright^{™} Plus Absolute Counting Beads, purchased from Invitrogen, C36995) were added to this cell suspension for absolute quantification of cancer cells remaining per treatment, analyzed via flow cytometry. Cytotoxicity results for each group was calculated according to the following equation: cytotoxicity (%) = (number of the target cells in target cell only group - number of target cells in co-culture group) / number of the target cells in target only group × 100%.

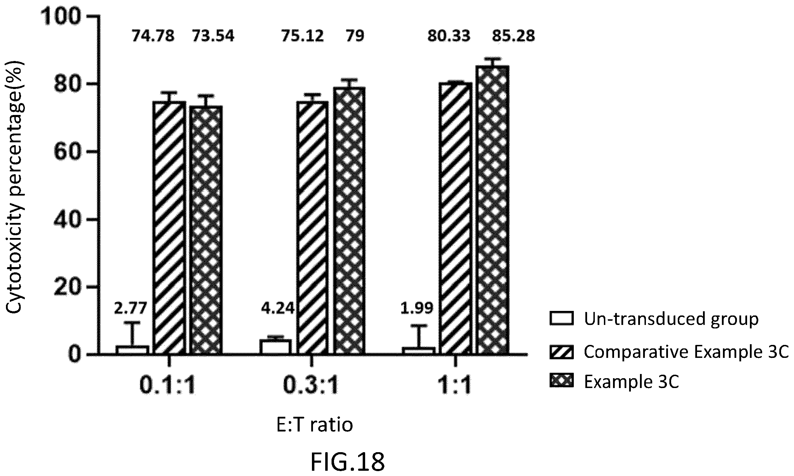

As shown in Fig. 18, when the E:T ratio was 0.1:1, the cytotoxicity of Example 3C was 73.54%, which was similar to that of Comparative Example 3C, which was 74.78%; whereas, when the E:T ratios were 0.3:1 and 1:1, the cytotoxicity of Example 3C were approximately 80% to 85%, which were both better than those of Comparative Example 3C at corresponding E:T ratios.

### Test Example 18: Real-time cytotoxicity assay for the analyses of Example 3C and Comparative Example 3C against H929 cancer cells

Human BCMA fragment was cloned from H929 cell line (purchased from BCRC, 60386). The cDNA fragment encoding truncated BCMA (shown as SEQ ID NO: 97) was amplified by PCR; wherein, the foresaid truncated BCMA cDNA fragment (amino acid 1 to 98) indicated cDNA fragment having extracellular and transmembrane domains but lacking intracellular domain and cytoplasmic signaling domains. The foresaid truncated BCMA cDNA fragment was ligated into pLAS5w.Ppuro vector (purchased from Academia Sinica RNAi Core facility, Product No.: C6-8-39), using restriction enzyme sites of HpaI and NheI, which following completion was then transfected into SKOV3 cells (human ovarian cancer cell line, purchased from ATCC, HTB-77) by applying Polyjet transfection reagent (purchased from SignaGen Laboratories, SL100688). The truncated BCMA transfected SKOV3 cells were cultured in McCoy 5A Medium (purchased from Gibco) containing 2 µg/mL of puromycin and 10% FBS to select a SKOV3 cell line that was puromycin-resistant and expressed BCMA at the same time. At last, the expression of BCMA was re-checked by flow cytometric analysis. The SKOV3 cell line expressing BCMA was used as target cells for the real-time cytotoxicity assay conducted using the xCELLigence system.

The method of Test Example 18 was similar to that of Test Example 6, except that the effector cells were CAR-T cells of Example 3C or Comparative Example 3C. The target cells were the foresaid SKOV3 cells expressing BCMA, which were seeded at a cell density of 1.2×10⁴ cells/mL into a 96-well microplate (E-plate, Agilent Technologies, 300600910) with its background impedance corrected, and left overnight to ensure that the target cells attached. To allow sufficient cancer cell growth, the background impedance measurement was performed for a total of 22 hours, at which time, the effector cells were then added into the microplate for co-culture at E:T ratios of 0.3:1 and 0.1:1.

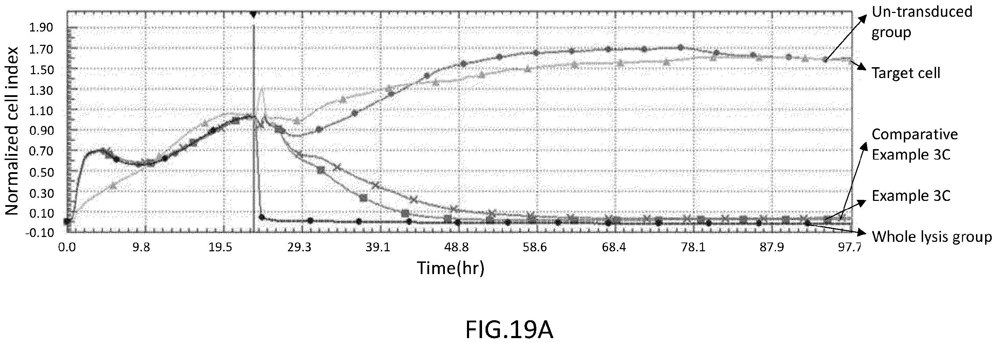

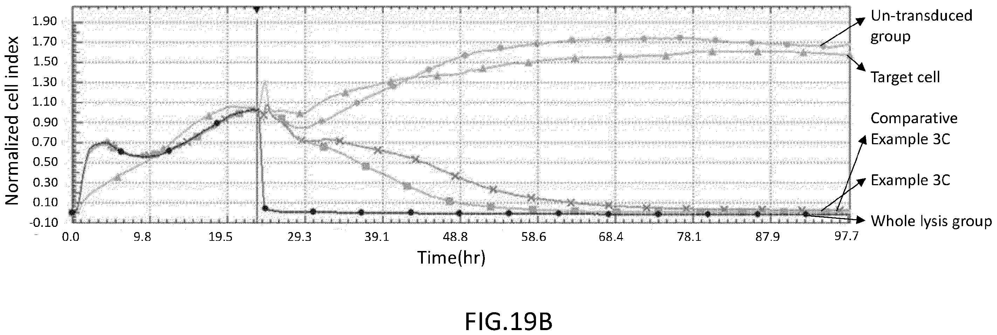

The experimental results of E:T ratios at 0.3:1 and 0.1:1 are shown in Fig. 19A and Fig. 19B, respectively, and the results of these figures showed that after co-culturing for 36 hours, which is also 58 hours from the start of experiment, CAR-T cells of Example 3C killed 100% of the SKOV3 cells expressing BCMA at both E:T ratios. The normalized cell index curve of Example 3C appeared nearly identical to that of the whole lysis group, indicating that the cytotoxicity of CAR-T cells of Example 3C was slightly stronger than that of the single-chain CAR-T cells of Comparative Example 3C.

In summary, the lentivirus of Example 3B (which had the hinge domain of CD28) had moderate to high transduction efficiency, and CAR-T cells of Example 3C expanded well, exhibited excellent cytotoxicity and higher proportions of Tscm, which could potentially lead to the reduction in cytokine release syndrome and neurotoxicity caused by such therapy. Therefore, the hinge domain of CD28 was a suitable hinge domain to match with the anti-BCMA receptor.

## Claims

1. An isolated nucleic acid molecule, **characterized by** comprising nucleic acid sequences encoding the following parts:
(a) an extracellular antigen binding domain, which comprises a heavy chain variable region;
(b) a hinge domain;
(c) a NKp30 transmembrane domain; and
(d) a NKp30 cytoplasmic domain.

2. The isolated nucleic acid molecule as claimed in claim 1, **characterized in that** the extracellular antigen binding domain comprises a heavy chain variable region of anti-mesothelin antibody, a heavy chain variable region of anti-CD22 antibody, a heavy chain variable region of anti-CD19 antibody, a heavy chain variable region of anti-BCMA antibody, a heavy chain variable region of anti-CD123 antibody, a heavy chain variable region of anti-GPRC5D antibody or a heavy chain variable region of anti-TSHR antibody.

3. The isolated nucleic acid molecule as claimed in claim 1 or 2, **characterized in that** the hinge domain is selected from the group consisting of: (G₄S)₃, a hinge domain of CD8, a hinge domain of CD28, a hinge domain of IgG4, EAAAKGGGGS, (EAAAK)₃, a hinge domain of GST, a hinge domain of EAAAK-GS, a hinge domain of IgD, and an IgG4-CH3 domain and (AP)₆.

4. The isolated nucleic acid molecule as claimed in any one of claims 1 to 3, **characterized in that** the isolated nucleic acid molecule further comprises a nucleic acid sequence encoding an adapter.

5. The isolated nucleic acid molecule as claimed in any one of claims 1 to 4, **characterized in that** the hinge domain and the NKp30 transmembrane domain are linked by a NKp30 stalk domain.

6. The isolated nucleic acid molecule as claimed in any one of claims 1 to 5, **characterized in that** the extracellular antigen binding domain comprises a heavy chain variable region of anti-mesothelin antibody, and the hinge domain is (G₄S)₃.

7. A plasmid, **characterized by** comprising the isolated nucleic acid molecule as claimed in any one of claims 1 to 6, wherein the plasmid is a DNA plasmid or an RNA plasmid.

8. A method for preparing a chimeric antigen receptor cell, **characterized in that** the method comprising introducing the isolated nucleic acid molecule as claimed in any one of claims 1 to 6 into a nucleated cell.

9. The method as claimed in claim 8, **characterized in that** the isolated nucleic acid molecule is introduced into the nucleated cell by lentivirus transduction.

10. An isolated cell, **characterized by** comprising the isolated nucleic acid molecule as claimed in any one of claims 1 to 6.

11. The isolated cell as claimed in claim 10, **characterized in that** the isolated cell comprises a T cell, a natural killer cell, a natural killer T cell or an adipose-derived stem cell.

12. A pharmaceutical composition, **characterized in that** the pharmaceutical composition is for treating or alleviating a cancer, and comprises an effective amount of the isolated cell as claimed in claim 10 or 11.

13. A pharmaceutical composition, **characterized in that** the pharmaceutical composition is for treating or alleviating an autoimmune disease, and comprises an effective amount of the isolated cell as claimed in claim 10 or 11; wherein the autoimmune disease comprises systemic lupus erythematosus, idiopathic inflammatory myositis, systemic sclerosis or multiple sclerosis.
